(19) Europäisches Patentamt
European Patent Office
Office européen des brevets

(11) EP 2 832 442 B1

(12) **EUROPEAN PATENT SPECIFICATION**

(45) Date of publication and mention of the grant of the patent:
**15.01.2020 Bulletin 2020/03**

(51) Int Cl.:
***B01J 13/14*** *(2006.01)* ***C11D 3/50*** *(2006.01)*

(21) Application number: **14178733.3**

(22) Date of filing: **28.07.2014**

(54) **MICROCAPSULES**

MIKROKAPSELN

MICROCAPSULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.07.2013 EP 13306096**

(43) Date of publication of application:
**04.02.2015 Bulletin 2015/06**

(73) Proprietor: **Takasago International Corporation
Tokyo 144-8721 (JP)**

(72) Inventors:
- **Warr, Jonathan
75017 Paris (FR)**
- **Ribaut, Tiphaine
75017 Paris (FR)**
- **Anthony, Olivier
75017 Paris (FR)**
- **Fraser, Stuart
Little Neston, Cheshire CH64 4DH (GB)**

(74) Representative: **Cabinet Beau de Loménie
158, rue de l'Université
75340 Paris Cedex 07 (FR)**

(56) References cited:
**EP-A1- 1 321 182**
**WO-A1-2012/110443**
**WO-A2-2009/037482**
**WO-A2-2009/077525**
**WO-A2-2010/145993**
**DE-A1- 19 749 731**
**DE-A1-102007 055 813**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

## Field of the Invention

The present disclosure discloses an aqueous dispersion including microcapsules which comprise a perfume composition enclosed within a polymeric shell, a process for the manufacture of that dispersion as well as non-ingestible consumer products (such as household cleaners, laundry products, personal care products and cosmetic products) containing that dispersion.

## Background

Microencapsulation represents a common solution to protect (e.g. upon storage) and control the delivery of hydrophobic materials such as fragrances. Microencapsulation of fragrances through free radical polymerization entails the preliminary formation of an emulsion wherein a continuous, typically aqueous-based, phase disperses an internal, hydrophobic phase containing the fragrances. By triggering polymerization within the emulsion, an aqueous dispersion of microcapsules is typically obtained wherein the microcapsules include the fragrances enclosed within a polymeric shell. The dispersion may then be incorporated into a final product such as a non-edible consumer goods product, a laundry product, a personal care product or a cosmetic product. Existing microencapsulation techniques are not always satisfactory when targeting effective encapsulation of high amounts of fragrances at affordable process costs. In effect, attempts to encapsulate high fragrance loadings with common and cost affordable techniques may fail or ultimately lead to aqueous dispersions of unsatisfactory quality. WO 2010/145993 discloses a process for preparing a microcapsule, the process comprising: polymerizing, in a the free-radical polymerization, an oil-in-water emulsion, wherein the oil-in-water emulsion comprises: (I) at least one monomer selected from the group consisting of a $C_1$-$C_{24}$-alkyl ester of acrylic acid or of methacrylic acid or of both; acrylic acid; methacrylic acid; maleic acid; fumaric acid; and itaconic acid, (II) a monomer of an ethylenically unsaturated crosslinker, comprising a highly branched polymeric crosslinker, (III) optionally, a monounsaturated monomer, which is different from monomer (I), and (IV) a hydrophobic core material, and wherein a total content of all monomer (I) based on a total weight of the monomers (I), (II), and (III) is from 30 to 90%, a total content of all monomer (II) based on the total weight of the monomers is from 10% to 70%, a total content of all monomer (III) based on the total weight of the monomers is from 0 to 30%, and a content of highly branched polymeric crosslinker based on the total weight of monomers is at least 10%.

## Summary

The present disclosure discloses an aqueous dispersion of fragrance-containing, polymeric microcapsules. The dispersions presently disclosed contain high fragrance loadings while maintaining an appropriate ease of processing. Also, an optimal delivery of fragrances is achieved as the microcapsules included in the dispersion may show advantageous stability properties such as reduced fragrance leakage for example upon storage and especially upon storage in a liquid medium. The microcapsules may also display pH-independent shell properties. This means for example that the microcapsules may display satisfactory shell stability in acid (e.g. from pH 2) and alkaline (e.g. up to pH 12) conditions as can be found in many liquid household, laundry personal care and cosmetic products, such as fabric conditioners and antiperspirants (acidic pH) or liquid laundry detergents and hard surface cleaners (alkaline pH). The instant disclosure also discloses a simple and effective polymerization process for the manufacture of an aqueous dispersion of fragrance-containing microcapsules. The instant disclosure also discloses a non-edible consumer goods product, a laundry product, a personal care product or a cosmetic product containing an aqueous dispersion of microcapsule as presently defined. In particular, the present disclosure discloses the following points:

1. An aqueous dispersion including a plurality of microcapsules, each microcapsule comprising a perfume composition enclosed within a polymeric shell, wherein

- the perfume composition includes a fragrance,
- the shell includes solid colloidal particles having an average primary particle size comprised between 5nm (nanometer) and 1$\mu$m (micrometer),
- the shell further includes in polymerized form a monomer blend including:

    i) between 30% and 80% by weight over the combined weight of compounds (I) to (II) in the blend of a compound (I) which is a monoethylenically unsatured monomer and/or dimethyldiallyl ammonium chloride (DMDAAC),

    ii) between 20% and 70% by weight over the combined weight of compounds (I) to (II) in the blend of a

compound (II) which is a polyethylenically unsatured monomer, wherein compound (II) is a $C_2$-$C_{24}$ alkyl di- or polyester of (meth)acrylic acid and:

A1. contains two or more (meth)acrylate ester groups per monomer, and
B1. has a molecular weight which, once divided by the number of (meth)acrylate ester groups, gives a value of more than 85 g/mol and lower than 135 g/mol;

wherein the combined amounts of compounds (I) and (II) make up 100% by weight of the blend, and
wherein the fragrance represents between 20% and 45% by weight over the weight of the dispersion.

2. The dispersion according to point 1, wherein the fragrance represents between 30% and 45% by weight over the weight of the dispersion.

3. The dispersion according to any one or more of points 1 to 2, wherein compound (I) is selected from (meth)acrylate monomers which are polymerizable through free-radical polymerization.

4. The dispersion according to any one or more of points 1 to 3, wherein compound (I) is selected the group consisting of methacrylic acid, methyl methacrylate, ethyl methacrylate, isopropyl methacrylate, 2-hydroxyethyl methacrylate, 2-hydroxypropyl methacrylate, 3-hydroxypropyl methacrylate and mixtures thereof.

5. The dispersion according to any one or more of points 1 to 2, wherein compound (I) is a combination of:

ia) between 50% and 100% by weight over the weight of the combination of a neutral monomethacrylate monomer (Ia) having a solubility in water at pH 7 and 20ºC equal to, or more of 2g/100ml,
ib) between 0% and 50% by weight over the weight of the combination of another neutral monoethylenically unsatured monomer (Ib), and
ic) between 0% and 15% by weight over the weight of the combination of a ionized or ionizable monoethylenically unsatured monomer (Ic).

6. The dispersion according to point 5, wherein the neutral monomethacrylate monomer (Ia) is selected from the group consisting of 2-hydroxyethyl methacrylate, 2-hydroxypropyl methacrylate, 3-hydroxypropyl methacrylate, glycidyl methacrylate, poly(ethylene glycol) methyl ether methacrylate and mixtures thereof.

7. The dispersion according to any one or more of points 1 to 6, wherein compound (II) is a di- or polyester resulting from the esterification of (meth)acrylic acid with a linear or branched polyhydric $C_2$-$C_{24}$ alcohol and/or $C_2$-$C_{24}$ polyethylene glycols.

8. The dispersion according to point 7, wherein compound (II) comprises one or more of 1,4-butylene glycol dimethacrylate, ethylene glycol dimethacrylate and 1,3-propylene glycol dimethacrylate.

9. A product comprising the dispersion as defined in any one or more of points 1 to 8.

10. A process for the manufacture of an aqueous dispersion as defined in any one or more of points 1 to 8, said process comprising the following steps:

a) providing an oil-in-water emulsion having an oil phase and a water phase, said emulsion being obtainable by mixing:

◦ solid colloidal particles having an average primary particle size comprised between 5nm and 1μm,
◦ a polymerization initiator,
◦ a perfume composition including a fragrance,
◦ an emulsifier, and
◦ a monomer blend as defined in any one or more of points 1 to 8,

b) triggering polymerization within the emulsion obtained in step a),
c) letting the polymerization propagate thereby obtaining microcapsules;

wherein the fragrance represents between 20% and 70%, for example between 20 and 45% by weight of the weight

of the emulsion.

## Detailed Description

Unless otherwise stated, all percentages are weight percentages.

Unless otherwise indicated "an" or "a" means one or more.

Unless otherwise indicated, all chemical terms have the meanings defined by the IUPAC Compendium of Chemical Terminology 2nd Edition Compiled by A D McNaught and A Wilkinson Blackwell Scientific Publications Oxford 1997 and IUPAC Nomenclature of Organic Chemistry, published by Blackwell Scientific Publications Oxford 1993 ISBN 0632034882.

Unless otherwise indicated, the language "blend", "a blend" or "monomer blend" refers to the blend including compounds (I) to (II).

Unless otherwise indicated, "monomer" means monomers which can be polymerized by free radical polymerization.

Unless otherwise indicated "(meth)acrylate" (or "(meth)acrylic") means methacrylate (or methacrylic) and/or acrylate (or acrylic). For example, it means methacrylate (or methacrylic). For example it means acrylate (or acrylic). For example it means methacrylate (or methacrylic) and acrylate (or acrylic).

Unless otherwise indicated, methacrylate and acrylate ester groups are groups having molecular weight of 85 and 71 mass units, respectively, and the following structures

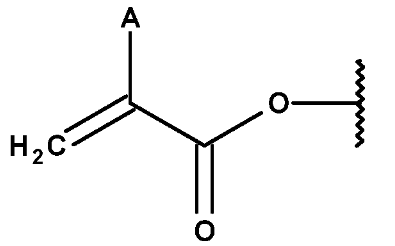

wherein A is $CH_3$ for a methacrylate ester group or A is H for an acrylate ester group.

Unless otherwise indicated, methacrylate or acrylate amide groups are groups having molecular weight of 84 and 70 mass units, respectively, and the following structures

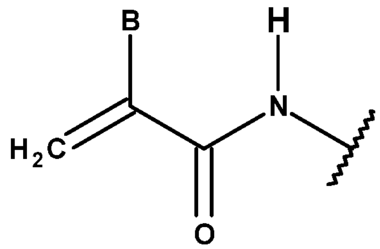

wherein B is $CH_3$ for a methacrylate amide group or B is H for an acrylate amide group.

Unless otherwise indicated, room temperature is 20ºC.

Certain substances, notably perfumery molecules, may exist as distinct isomers (or as mixture of distinct isomers). Hereinafter, they may be identified also by means of their CAS number. In these cases, the CAS number of a single isomer is reported. However, and unless otherwise indicated, the reference shall be understood to cover all existing isomers.

The present disclosure discloses an aqueous dispersion including a plurality of microcapsules, each microcapsule including, such as consisting of, a perfume composition enclosed within a polymeric shell. Terms such as "slurry" or "slurry dispersion" may also be used hereinafter to refer to the dispersion.

The dispersion may comprise, for example essentially consist of, a water-based liquid medium (i.e. the dispersing medium) and a plurality of solid microcapsules dispersed in the medium. Traces of other ingredients used in the manufacturing process (such as polymerization initiators and residual, unreacted monomers) may also be present.

In the dispersion, the fragrance typically represents between 20% and 45%, such as more than 25%, for example more than 30% or more than 33% and less than 40%, for example less than 35% by weight of the weight of the dispersion.

The water-based liquid medium may include water, such as deionized water.

The dispersion may be the product directly obtainable by a free radical polymerization process as defined below.

The dispersion may conveniently be used to prepare e.g. liquid products that will be discussed later in this disclosure. The slurry functions thus as a concentrated fluid which is added to the liquid products.

The slurry can also conveniently be used as a storage medium for the microcapsules of the invention. In case no water (or a limited amount of water) must be present in the final product, the slurry can be preliminary subjected to a spray-drying step and the spray-dried product is then added to the final intended product.

Methods of preparation of perfume-containing microcapsules are described for example in MICROENCAPSULATION: Methods and Industrial Applications Edited by Benita and Simon (Marcel Dekker, Inc. 1996) and in Kirk Othmer Encyclopedia of Chemical Technology Microencapsulation by C. Thies. Microcapsules obtainable by free radical polymerization are well known to those working in the field of e.g. encapsulated perfumes and are structurally (and dimensionally) different from other types of capsules such as conventional seamless soft capsules or two-piece hard capsules used e.g. in pharmacy to orally or rectally administrate substances to a subject.

The microcapsules presently disclosed are not intended for oral or rectal administration to human or animal subjects.

A microcapsule as presently disclosed may have a shell thickness comprised between about 100nm and 800nm, such as between about 200nm and 700nm, for example between about 300nm and 600nm.

The microcapsules as presently disclosed may have a perfume composition-to-shell weight ratio which is comprised between 50:1 and 1:1, such as between 30:1 and 1:1, or between 20:1 and 1:1, for example between 10:1 and 1:1.

The microcapsules presently disclosed may be substantially spherical.

The microcapsules presently disclosed may have an average particle size (median volume particle size D(v; 0.5) value) equal to or greater than 7.5 microns (7.5μm), for example equal to or greater than 10μm, such as equal to or greater than 15μm, or equal to or greater than 20μm, for example equal to or greater than 25μm. The microcapsule presently disclosed may have an average particle size equal to or less than 60microns (60μm), for example equal to or less than 50μm, such as equal to or less than 45μm, for example equal to or less than 40μm. The microcapsule presently disclosed may have an average particle size comprised between 7.5 microns (7.5μm) and 60 microns (60μm), or between 7.5μm and 50μm, or between 10μm and 50μm, or between 7.5μm and 45μm, or between 10μm and 45μm, or between 15μm and 45μm, or between 15μm and 40μm, or between 20μm and 45μm, or between 25μm and 45μm, or between 25μm and 40μm, or between 25μm and 35μm.

Microcapsules obtainable by free-radical polymerization have typically quite small (e.g. less than about 7 microns) average particle sizes. This might be due to a technical belief that this size better copes with an efficient polymerization, thus leading to capsules with better properties. At the same time, it was also believed that average particle size did not have a significant impact on final capsule leakage. The experimental results obtained by the present Applicant showed however that no significant issues with polymerization are met when targeting larger sizes and that larger average particle sizes may bring about an advantage in terms of leakage. If microcapsules with dimensions which do not make them visible at naked eye when deposited on a black surface are desired, then it is recommendable to target an average particle size of less than e.g. 70 microns.

The preferred technique used in the present disclosure to measure the microcapsule average particle size is light scattering using for example a Horiba® or a Malvern® Laser scattering particle Size Distribution analyzer or an equivalent instrument working on the principle of low angle laser light scattering (LALLS) following the general guidelines set out in ISO 13320 "Particle Size Analysis - Laser Diffraction Methods".

The microcapsule polymeric shell comprises solid colloidal particles (also known as particulate colloids) having an average primary particle size comprised between 5nm and 1μm as measured for example through dynamic light scattering. Free radical polymerization for microcapsule preparation generally includes the initial formation of an oil-in-water emulsion. Particulate colloids allow obtaining Pickering oil-in-water emulsions stabilized by limited coalescence. The process of formation of Pickering emulsions is known. It is discussed for example in Whitesides and Ross, J. Interface Colloid Sci. 196, 48-59(1995).

Examples of materials which can be suitably used in the form of solid colloidal particles in the microcapsules presently disclosed are silica, quartz, glass, aluminum (AlO(OH)), alumino-silicates (e.g. clays), silicon, copper, tin (SnO), talc, inorganic oxides or hydroxides (e.g. $Fe_2O_3$, $TiO_2$, $Cr_2O_3$), steel, iron, asbestos, nickel, zinc, lead, marble, chalk ($CaCO_3$), gypsum ($CaSO_4$), barytes (e.g. $BaSO_4$), graphite and carbon black. Preferred materials are silica, alumino-silicates and inorganic oxides or hydroxides. Silica is a highly preferred material.

Solid colloidal particles suitable for the present disclosure may or may not be surface modified. Surface modification may either impart the ability to materials to partition to the interface of water and oil phases or it may improve the compatibility between the materials and the microcapsule polymeric shell. Examples of surface modification include chemical treatments to increase or decrease particles hydrophobicity. Alternatively, surface modifying agents can be adsorbed onto particles surface to impart appropriate surface active properties. Alternatively, particles may be modified by means of coupling agents which improve the compatibility between the materials and the microcapsule polymeric shell. Techniques to modify particle surfaces are discussed for example in "Nanoparticle Technology handbook" 1st

edition, year 2007, Application 41 (pages 593-596) "Surface modification of inorganic nanoparticles by organic functional groups". Modified (as well as non-modified) solid colloidal particles are commercially available.

Examples of suitable colloidal silicas may be dry fumed silicas (such as commercially available in the Aerosil® range from Evonik®) or aqueous colloidal silica dispersions (such as those commercially available in the Ludox® range from Du Pont®). Dry silica particles may be fumed silica particles or condensed silica particles. Fumed silicas are particularly adapted for stabilizing emulsions with droplet sizes in the range of 10μm to 100μm. For larger droplets, colloidal silicas might be more appropriate. Suitable grades of fumed silica are Aerosil® 200 (a hydrophilic fumed silica with a specific surface area of 200 $m^2/g$) and Aerosil® R816 having a BET surface area of 190 ± 20$m^2/g$ and an average primary particle size of about 12 nm, both available from Evonik®.

Amounts of solid colloidal particles may be comprised between 0.005% and 10%, such as between 0.01% and 5%, for example between 0.02% and 3%, such as between 0.05% and 2%, or between 0.1% and 1%, such as 0.25% by weight over the weight of the plurality of microcapsules.

The use of solid colloidal particles as presently defined has been found to permit a satisfactory and effective microencapsulation of desirably high fragrance loadings while maintaining a suitable quality of the resulting dispersion (e.g. handling quality, permitting a good processability of the dispersion). This allows encapsulating more fragrance than usual while not being confronted with insurmountable manufacturing hurdles or having to tolerate a non-ideal dispersion quality. These results are achievable without relying on complex and expensive manufacturing techniques such as high shearing during the entire process, especially during polymerization.

Although it is not intended to be bound by any theory, advantages of the claimed use may be experimentally confirmed by primarily noting that viscosity values measured in the instant aqueous dispersions are generally improved with respect to the corresponding values as measured in identical measuring conditions, in identical aqueous dispersions obtainable in identical conditions but without solid colloidal particles. Improvements in term of viscosity are also generally mirrored by corresponding improvements in terms of span ratio. The Span ratio and/or viscosity may thus be valuable tools to evaluate the aqueous dispersion quality. In effect, they reflect the amount of (unwanted) generally very small latex particles that form in the water phase during polymerization while also providing an indication of microcapsule size uniformity and microcapsule amount in the dispersion.

Identical conditions are preferably identical qualitative and quantitative conditions such as identical fragrance loading and fragrance chemical nature, identical monomer blend, identical process conditions and identical process ingredients.

Compound (I) is preferably a monoethylenically unsatured monomer.

Examples of suitable monoethylenically unsatured monomer polymerizable by a free radical polymerization are:

- alpha-olefins;
- dienes such as butadiene;
- styrene, alpha-methyl styrene, sodium 4-styrene sulfonate;
- vinyl ethers, such as ethyl vinyl ether, chloroethyl vinyl ether, vinyltrimethylsilane;
- vinyl ketones, such as methyl vinyl ketone;
- vinyl naphthalene;
- 4-vinylbenzoic acid;
- allyl and vinyl chloroacetate;
- vinyl halides and vinylidene halides, for example, vinyl bromide, vinylidene chloride
- vinyl acetate;
- 2-vinyl pyridine and 4-vinyl pyridine;
- N-vinylpyrrolidone;
- acrylic acids and derivatives for example, acrylic acid, methacrylic acid, crotonic acid;
- itaconic acid and itaconate esters such as dibutyl itaconate;
- esters of acrylic acid, such as methyl acrylate, ethyl acrylate, propyl acrylate, butyl acrylate, isopropyl acrylate, tert-butyl acrylate, isobutyl acrylate, n-butyl acrylate, tetrahydrofurfuryl acrylate, octyl acrylate, octadecyl acrylate, Isooctyl acrylate, Isobutyl acrylate, isobornyl acrylate, lauryl acrylate, cyanoethyl acrylate, 2-hydroxyethyl acrylate, 2-ethylhexyl acrylate, 2-[[(butylamino)carbonyl]oxy]ethyl acrylate, acryloxyethyltrimethyl ammonium chloride, 3-Sulfopropyl acrylate potassium salt, Poly(propylene glycol) acrylate, Poly(ethylene glycol) diacrylate;
- esters of methacrylic acid, such as methyl methacrylate, ethyl methacrylate, propyl methacrylate, butyl methacrylate, isopropyl methacrylate, tert-butyl methacrylate, isobutyl methacrylate, n-butyl methacrylate, benzyl methacrylate, isobornyl methacrylate, cyclohexyl methacrylate, tetrahydrofurfuryl methacrylate, mono(ethylene glycol) methyl ether methacrylate, di(ethylene glycol) methyl ether methacrylate, 2-hydroxyethyl methacrylate, 2-hydroxypropyl methacrylate, 3-hydroxypropyl methacrylate, glycidyl methacrylate, triethylene glycol methyl ether methacrylate, poly(ethylene glycol) methyl ether methacrylate, 2-(diethylamino)ethyl methacrylate, dimethylaminoethyl methacrylate, 2-(tert-butylamino)ethyl methacrylate, 2-ethyl(2-oxoimidazolidin-1-yl)methacrylate, methacryloxyethyltrimethyl ammonium chloride, metracryloxypropyltrimethoxysilane, metracryloxypropyltriethoxysilane;

- nitriles, such as acrylonitrile, methacrylonitrile;
- acrylamides and methacrylamides, such as methyl acrylamide, 4-Acryloylmorpholine, N-methylol acrylamide, 2-acrylamido-2-methylpropane sulfonic acid, N-butoxy methacrylamide, N-(3-dimethylaminopropyl methacrylamide, dimethylaminopropyl methacrylamide, 3-trimethylammonium propyl methacrylamide chloride;
- alkyl crotonates, and related esters such as methyl crotonate;
- cyclic and polycyclic olefin compounds for example, cyclopentene, cyclohexene, cycloheptene, cyclooctene, and cyclic derivatives up to 20 carbon atoms; polycyclic derivates for example, norbornene, and similar derivatives up to 20 carbon atoms;
- cyclic vinyl ethers for example, 2,3-dihydrofuran, 3,4-dihydropyran, and similar derivatives;
- allylic alcohol derivatives for example, vinylethylene carbonate; and
- disubstituted olefins such as maleic and fumaric compounds for example, maleic anhydride, diethylfumarate.

In particular, suitable examples of compound (I) are:

a) $C_3$-$C_6$ monoethylenically unsatured mono- or poly carboxylic acids,
b) amides of $C_3$-$C_6$ monoethylenically unsatured mono- or poly carboxylic acids;
c) optionally mono- or polysubstituted $C_1$-$C_{24}$ linear or branched alkyl esters of $C_3$-$C_6$ monoethylenically unsatured mono- or poly carboxylic acids,
and
d) optionally mono- or polysubstituted $C_3$-$C_6$ cycloalkyl esters of $C_3$-$C_6$ monoethylenically unsatured mono- or poly carboxylic acids,

wherein optional substituents are selected from the group consisting of -OH, -OR, - C(O)R, $-NH_2$, -NHR, $-NR_2$, $-NR_3^+$, $-C_5$-$C_6$ aromatic or heteroaromatic rings, and $C_3$-$C_{10}$ cyclo- or heterocyclic alkyl,
wherein R is $C_1$-$C_4$ alkyl. The carboxylic acid is preferably a monocarboxylic acid such as methacrylic acid.

For example, compound (I) is selected from acrylic acid, methacrylic acid, maleic acid, itaconic acid, 2-(diethylamino)ethyl methacrylate, dimethylaminoethyl methacrylate, 2-(tert-butylamino)ethyl methacrylate, N-[3-(dimethylamino)propyl]methacrylamide, 3-trimethylammonium propyl methacrylamide chloride, methyl methacrylate, ethyl methacrylate, n-propyl methacrylate, isopropyl methacrylate, tert-butyl methacrylate, isobutyl methacrylate, n-butyl methacrylate, methacrylamide, benzyl methacrylate, isobornyl methacrylate, cyclohexyl methacrylate, tetrahydrofuryl methacrylate, glycidyl methacrylate, 2-hydroxyethyl methacrylate, 2-hydroxypropyl methacrylate, 3-hydroxypropyl methacrylate, poly(ethylene glycol) methyl ether methacrylate, 2-ethyl(2-oxoimidazolidin-1-yl)methacrylate, acryloxyethyltrimethyl ammonium chloride, methacryloxyethyltrimethyl ammonium chloride and mixtures thereof.

Compound (I) may be selected from methacrylic acid, methyl methacrylate, ethyl methacrylate, isopropyl methacrylate, 2-hydroxyethyl methacrylate, 2- or 3-hydroxypropyl methacrylate and mixtures thereof, for example methacrylic acid, methyl methacrylate, ethyl methacrylate and mixtures thereof.

Compound (I) may be a combination of methacrylic acid and methyl and/or ethyl methacrylate. The combination may be used in an amount comprised between 30% and 60%, for example between 35% and 60% by weight over the combined weight of compounds (I) to (II) in the blend. In the combination, methacrylic acid may be present between 35% and 50%, such as between 45 and 47%, by weight, and methyl or ethyl methacrylate between 0% and 15%, such as between 0 and 8%, by weight over the combined weight of compounds (I) to (II) in the blend.

Advantageously, compound (I) is a monomethacrylate unsatured monomer meaning that it contains one single methacrylate ester group. In effect, methacrylates proved to be less susceptible than acrylates to hydrolysis on prolonged exposure to acidic or alkaline pH and elevated storage temperatures. Hence, it may be advantageous that compound (I) does not contain acrylic acid derivatives such as $C_1$-$C_{24}$ alkyl or $C_3$-$C_6$ cycloalkyl esters or amides of acrylic acid.

In one embodiment, the monomer blend includes for example between 30% and 60%, for example between 35% and 60% by weight over the combined weight of compounds (I) and (II) in the blend of a compound (I) which is a combination of:

ia) between 50% and 100%, such as between 60% and 100%, for example between 70% and 100% by weight over the weight of the combination of a neutral monomethacrylate monomer (Ia) having a solubility in water at 20ºC equal to, or more of 2g/100ml,
ib) between 0% and 50%, such as between 0% and 60%, for example between 0% and 30% by weight over the weight of the combination of another neutral monoethylenically unsatured monomer (Ib), and
ic) between 0% and 15%, such as between 0% and 5% by weight over the weight of the combination of a ionized or ionizable monoethylenically unsatured monomer (Ic).

Adopting the above combination of monomers (Ia) to (Ic) allows obtaining microcapsules which display shell properties which are pH-independent in a pH range commonly met in liquid household, laundry personal care and cosmetic products, such as fabric conditioners and antiperspirants (acidic pH) or liquid laundry detergents and hard surface cleaners (alkaline pH). For example, this pH range is comprised between 2 and 12, such as more than 4, for example between 4 and 12. Hence, it is particularly advantageous that a product (as defined below) including a microcapsule obtainable with that blend is liquid at room temperature and has a pH of, for example, more than 4, such as more than 4 and less than 12.

In the present description and unless otherwise indicated, "neutral" means that the monomethacrylate monomer is non-ionized or ionized in an amount of less than 20 mol% when measured in deionized water at 20ºC at a pH of 2 and 12. For example, a monomethacrylate monomer is neutral if it does not contain functional groups which are permanently ionized such as quaternized amines, for example quaternary alkyl ammonium salts. For example, a neutral monomethacrylate monomer may contain functional groups whose protonated species have $pK_a$ greater than about 12.5, such as greater than about 12.7, for example greater than about 13, such as comprised between about 13 and 30. For example, a monomethacrylate monomer is neutral if it does not contain functional groups such as carboxylic acid groups, primary or secondary amine groups. Alternatively, a neutral monomethacrylate monomer may contain functional groups such as primary alcohols, primary or secondary amides or ether groups.

Monomer (Ia) has a solubility in water at pH 7 and 20ºC equal to, or more of 2g/100ml, for example more than 3, such as more than 4 or more than 5g/100ml. Monomer (Ia) is a hydrophilic one. Water solubility is conveniently measured according to OECD method 105 - water solubility adopted on 27 July 1995 (OECD GUIDELINE FOR THE TESTING OF CHEMICALS).

Monomer (Ia) may be selected from 2-hydroxyethyl methacrylate, 2-hydroxypropyl methacrylate, 3-hydroxypropyl methacrylate, methacrylamide, glycidyl methacrylate, methacrylonitrile, poly(ethylene glycol) methyl ether methacrylate, for example PEG300 methacrylate methyl ether or for example a poly(ethylene glycol) methyl ether methacrylate wherein the average number of PEG units is comprised between 3 and 20, for example between 5 and 10 (e.g. triethylene glycol methyl ether methacrylate; tetraethyleneglycol methyl ether methacrylate; penta ethyleneglycol methyl ether methacrylate; decaethyleneglycol methyl ether methacrylate; pentadecaethyleneglycol methyl ether methacrylate), and mixtures thereof. For example, monomer (Ia) may be selected from 2-hydroxyethyl methacrylate, 2-hydroxypropyl methacrylate, 3-hydroxypropyl methacrylate, glycidyl methacrylate, triethylene glycol methyl ether methacrylate; PEG300 methacrylate methyl ether, and mixtures thereof. For example, monomer (Ia) may be selected from 2-hydroxyethyl methacrylate, glycidyl methacrylate, poly(ethylene glycol) methyl ether methacrylate and mixtures thereof.

Preferably, monomer (Ia) includes at least 2-hydroxyethyl methacrylate. For example, 2-hydroxyethyl methacrylate may represent at least 10% or at least 20% or at least 30% or at least 40% or at least 50% or at least 60% or at least 70% or at least 80% or at least 90% by weight of the monomer (Ia) in the blend. Monomer (Ia) may consist of 2-hydroxyethyl methacrylate.

Monomer (Ib) is a neutral monoethylenically unsatured monomer other than, i.e. different from monomer (Ia). Neutral is defined as discussed above.

Suitable examples of monomers (Ib) may be:

- optionally substituted $C_1$-$C_{24}$ linear or branched alkyl esters of $C_3$-$C_6$ monoethylenically unsatured mono- or poly carboxylic acids,
  and
- optionally substituted $C_3$-$C_6$ cycloalkyl esters of $C_3$-$C_6$ monoethylenically unsatured mono- or poly carboxylic acids.

Optional substituents may be -OH, -OR, -C(O)R, wherein R is $C_{1}$-$C_4$ alkyl while a preferred mono- or poly carboxylic acid is methacrylic acid.

Monomer (Ib) may conveniently have a solubility in water at pH 7 and 20ºC of less than 2g/100ml. It may be totally insoluble in water. Monomer (Ib) is a hydrophobic one. Water solubility is conveniently measured according to OECD method 105 - water solubility adopted on 27 July 1995 (OECD GUIDELINE FOR THE TESTING OF CHEMICALS).

Monomer (Ib) may be selected from methyl methacrylate, ethyl methacrylate, n-propyl methacrylate, isopropyl methacrylate, tert-butyl methacrylate, isobutyl methacrylate, n-butyl methacrylate, benzyl methacrylate, isobornyl methacrylate, cyclohexyl methacrylate, tetrahydrofurfuryl methacrylate, mono(ethylene glycol) methyl ether methacrylate, di(ethylene glycol) methyl ether methacrylate, and mixtures thereof. For example, monomer (Ib) may be selected from methyl methacrylate and/or ethyl methacrylate.

Preferably, monomer (Ib) includes at least methyl methacrylate. Preferably, monomer (Ib) includes at least ethyl methacrylate. For example, methyl methacrylate and/or ethyl methacrylate may be present in an amount of at least 10%, such as at least 20%, for example at least 30%, such as at least 40%, or at least 50%, or at least 60%, or at least 70%, such as at least 80%, for example at least 90% by weight over the combined weight of all monomers (Ib) present in the blend. Monomer (Ib) may consist of methyl methacrylate and/or ethyl methacrylate.

Monomer (Ic) is a ionized or ionizable monoethylenically unsatured monomer.

In the present description and unless otherwise indicated, "ionized or ionizable" means that monomer (Ic) is either permanently ionized or ionized in an amount of more than 20 mol% when measured in deionized water at 20ºC at a pH of either2 or 12. For example, monomer (Ic) is ionized or ionizable if it contains functional groups which are permanently ionized such as quaternized amines, for example quaternary alkyl ammonium salts. For example, monomer (Ic) may contain functional groups whose protonated species have $pK_a$ lower than about 12.5, such as lower than about 11, for example lower than about 10, such as comprised between about 10 and 0. For example, a ionized or ionizable monomer (Ic) may contain one or more of functional groups such as carboxylic acid groups, sulfonic acid groups and primary or secondary amine groups.

Examples of monomer (Ic) are (meth)acrylic acid, 3-(methacryloylamino)propyl]trimethylammonium chloride, dimethyldiallyl ammonium chloride, maleic acid, itaconic acid, 2-(diethylamino)ethyl methacrylate, dimethylaminoethyl methacrylate, 2-(tert-Butylamino)ethyl methacrylate, N-[3-(dimethylamino)propyl]methacrylamide, acryloxyethyltrimethyl ammonium chloride, 2-ethyl(2-oxoimidazolidin-1-yl)methacrylate and mixtures thereof. Preferred examples are methacrylic acid and/or 3-(methacryloylamino)propyl]trimethylammonium chloride. Compound (II) may also be referred to as crosslinker due its crosslinking function in the manufacturing of the capsule shell.

Compound (II) is a polyethylenically unsatured monomer.

Examples of polyethylenically unsatured monomer polymerizable by a free radical polymerization are:

- polyacrylic esters of polyols, such as ethylene glycol diacrylate 1,4-butane diol diacrylate, 1,6-hexane diol diacrylate, trimethylol propane triacrylate, pentaerythritol tetraacrylate;
- polymethacrylic esters of polyols, such as ethylene glycol dimethacrylate, 1,3-propylene glycol dimethacrylate, 1,4-butane diol dimethacrylate, 1,6-hexane diol dimethacrylate, trimethylol propane trimethacrylate, ethoxylated pentaerythritol tetramethacrylate (MW about 585).

Compound (II) is a $C_2$-$C_{24}$ alkyl di- or polyester of (meth)acrylic acid, and:

A1. It contains two or more, for example 2 to 6, or 2 to 4 such as 2 or 3 or 4 (meth)acrylate ester groups per monomer, and

B1. It has a MW (molecular weight, expressed as mass units) which, once divided by the number of (meth)acrylate ester groups, gives a value of more than about 85, for example more than about 90, and lower than about 135, such as lower than about 121.

Preferably, compound (II) is a $C_2$-$C_{24}$ alkyl di- or polyester of methacrylic acid.

Suitable di- or polyesters are those resulting from the esterification of methacrylic acid with linear or branched polyhydric $C_2$-$C_{24}$, such as $C_2$-$C_{12}$, alcohols or $C_2$-$C_{24}$, such as $C_2$-$C_{12}$, polyethylene glycols. Suitable polyhydric alcohols may be those having a number average molecular weight of up to about 6000. Suitable polyethylene glycols may be those having a number average molecular weight of up to about 7500. Polyhydric alcohols are advantageously diols. Polyethylene glycols are advantageously di-, tri- or tetraethylene glycols.

Examples of compound (II) are 1,4-butylene glycol dimethacrylate (molecular weight MW about 226); 1,3-butylene glycol dimethacrylate (MW about 226); pentaerythritol trimethacrylate (MW about 340); glycerol trimethacrylate (MW about 296); 1,2-propylene glycol dimethacrylate (MW about 212), 1,3-propylene glycol dimethacrylate (MW about 212), ethylene glycol dimethacrylate (MW about 198), diethylene glycol dimethacrylate (MW about 242); glycerol dimethacrylate (MW about 228); 1,6-hexane diol dimethacrylate (MW about 226), trimethylolpropane trimethacrylate (MW about 338); ethoxylated pentaerythritol tetramethacrylate (MW about 585), and mixtures thereof. Preferred examples are 1,4-butylene glycol dimethacrylate, 1,3-propylene glycol dimethacrylate, ethylene glycol dimethacrylate, diethylene glycol dimethacrylate and mixtures thereof, such as 1,4-butylene glycol dimethacrylate, ethylene glycol dimethacrylate, 1,3-propylene glycol dimethacrylate and mixtures thereof.

Compound (II) may include at least 1,4-butylene glycol dimethacrylate, 1,3-propylene glycol dimethacrylate, ethylene glycol dimethacrylate or diethylene glycol dimethacrylate, such as at least 1,4-butylene glycol dimethacrylate and/or ethylene glycol dimethacrylate and/or 1,3-propylene glycol dimethacrylate. For example, monomer (II) may include at least, or consist of, 1,4-butylene glycol dimethacrylate. For example, monomer (II) may include at least, or consist of, ethylene glycol dimethacrylate. For example, monomer (II) may include at least, or consist of, 1,3-propylene glycol dimethacrylate. For example, monomer (II) may include the above crosslinkers in an amount of at least 10%, such as at least 20%, for example at least 30%, such as at least 40%, or at least 50%, or at least 60%, or at least 70%, such as at least 80%, for example at least 90% by weight over the combined weight of compound (II) in the blend.

In one embodiment, compound (II) meets conditions A1 and B1 above provided that any one or more of 1,4-butylene glycol dimethacrylate, ethylene glycol dimethacrylate, 1,3-propylene glycol dimethacrylate, ethylene glycol dimethacrylate and diethylene glycol dimethacrylate are excluded.

Compound (II) may be present between 30 and 60%, or between 35 and 60%, or between 40 and 55% by weight over the combined weight of compounds (I) to (II).

In one embodiment, the aqueous dispersion includes a blend as presently defined wherein the blend includes less than 15% by weight over the weight of the blend of monomers (I) and (II) having water solubility at 20ºC greater than 20g/100ml and less than 60% by weight over the weight of the blend of monomers (I) and (II) having water solubility at 20ºC greater than 20g/100ml. Water solubility for a given monomer is measured at a pH at which that monomer is neutral (so as to avoid magnifying effect of ionization on water solubility). For example, neutral means a pH at which the given monomer is non-ionized or ionized in an amount of less than 20 mol% when measured in deionized water at 20ºC.

The monomer blend may include, such as consist of:

- between 30 and 60%, such as between 35 and 60% of compound (I), and
- between 20 and 70%, such as between 35 and 60% of compound (II)

over the combined weight of compounds (I) to (II).

For example, the shell comprises in polymerized form a monomer blend including, such as consisting of:

i) between 30% and 60% by weight over the combined weight of compounds (I) to (II) in the blend of a compound (I) which is a combination of methacrylic acid with methyl or ethyl methacrylate, and

ii) between 20% and 70%, preferably between 30% and 60% by weight over the combined weight of compounds (I) to (II) in the blend of a compound (II) which is a $C_2$-$C_{24}$ alkyl di- or polyester of methacrylic acid, such as a monomer selected from 1,4-butane diol dimethacrylate, ethylene glycol dimethacrylate, 1,3-propylene glycol dimethacrylate and mixtures thereof.

For example, the shell comprises in polymerized form a monomer blend including, preferably consisting of:

i) between 30% and 70%, such as between 30% and 60% by weight over the combined weight of compounds (I) to (II) in the blend of a compound (I) which is a combination of:

ia) between 70% and 100% by weight over the weight of the combination of 2-hydroxyethyl methacrylate;

ib) between 0% and 30% by weight over the weight of the combination of a $C_1$-$C_{24}$ linear or branched alkyl ester of methacrylic acid such as methyl and/or ethyl methacrylate;

ic) between 0% and 5% by weight over the weight of the combination of methacrylic acid and/or 3-(methacryloylamino)propyl]trimethylammonium chloride; and

ii) between 20% and 70%, such as between 30% and 60% by weight over the combined weight of compounds (I) to (II) in the blend of a compound (II) which is a $C_2$-$C_{24}$ alkyl di- or polyester of methacrylic acid, such as a monomer selected from 1,4-butane diol dimethacrylate, ethylene glycol dimethacrylate, 1,3-propylene glycol dimethacrylate and mixtures thereof.

The monomer blend consists of compounds (I) to (II) as presently defined, meaning that the combined amounts of compounds (I) to (II) make 100% of the weight of the blend.

The monomer blend is free of monoethylenically unsatured monomers other than compound (I) as presently defined.

The monomer blend is free of polyethylenically unsatured monomers other than compound (II) as presently defined.

The monomer blend is free of one or more of:

- monomers, such as acrylic acid, which contain carboxylic acid (-COOH) groups and/or primary or secondary amine groups, in either neutral or ionized form;
- $C_1$-$C_{24}$ alkyl monoesters of acrylic acid;
- $C_2$-$C_{24}$ alkyl poly (e.g. di-, tri-, tetra- or penta) esters of acrylic acid (crosslinkers);
- monomers containing a carboxyl anhydride group (e.g. a monomer containing symmetric or asymmetric intermolecular anhydrides of monoethylenically unsatured monocarboxylic acids having 3 to 20 carbon atoms);
- monomers containing alkylenebis(meth)acrylamide group (e.g. N,N'-unsubstituted $C_{1-18}$ alkylene bis(meth)acrylamides or linear or cyclic N,N'-substituted $C_{1-18}$ alkylene bis(meth)acrylamides wherein substituents are selected from $C_{1-8}$ alkyl, $C_{1-8}$ hydroxyalkyl or polyoxy($C_{1-4}$)alkylene of 2 to 500 alkylene units or the alkyl substituents together with the nitrogen atoms to which they are attached form a 5- to 8-membered ring).

The perfume composition includes, such as consists of, a fragrance, i.e. an olfactively active (i.e. odoriferous) material typically but not necessarily providing a pleasant smell.

The perfume composition presently disclosed may also include a perfumery acceptable solvent and/or a benefit agent. For example, provided that the condition defining the weight of the fragrance with respect to the weight of the dispersion is met, the fragrance may represent at least 40%, such as at least 60%, for example at least 80%, such as at least 90% by weight over the weight of the perfume composition, the balance being represented by perfumery acceptable solvents and/or benefit agents as defined below.

The fragrance may consist of a single, typically organic, molecule or a mixture of distinct molecules. Hereinafter, these molecules will also be referred to as "perfumery molecules". Fragrance typically used in the field of perfumery and suitable for the purposes of the present disclosure are described more fully in S. Arctander, Perfume Flavors and Chemicals 1969, Vols. I and II, Montclair, N.J and in Allured’s Flavor and Fragrance Materials 2007 ISBN 978-1-93263326-9 published by Allured Publishing Corp. The term fragrance comprises both naturally occurring as well as synthetic fragrances known for use in perfumes. Perfumery molecules advantageously display balanced volatility/hydrophobicity so as to be olfactively noticeable when the microcapsules release them but also sufficiently water-insoluble to be emulsified during encapsulation.

The perfume composition may comprise at least two, such as at least four, or at least eight distinct fragrances. Effectively encapsulating high loadings of complex fragrance mixtures is particularly challenging due to the chemical diversity of these mixtures. In effect, structural differences in the various perfumery molecules may bring about greater difficulties in performing an effective encapsulation and obtain aqueous dispersions endowed with a suitable quality.

For example a fragrance may comprise at least two distinct perfumery molecules whose combination does not display a solid-liquid phase transition at a temperature comprised between -20°C and 120°C.

A fragrance may comprise one or more distinct perfumery molecules each having a molecular weight greater than 100, preferably greater than 125 and lower than 325, preferably lower than 300, more preferably lower than 275. A fragrance may comprise one or more distinct perfumery molecules each having a boiling point comprised between about 80ºC and 400ºC, such as between about 100ºC and 350ºC when measured at 760 mm Hg. It is preferable that perfumery molecules have water solubility below 1.5g/100ml at 20ºC. It is possible for example that a fragrance according to the present disclosure contains at least 80% by weight over the weight of the fragrance of a perfumery molecule as defined above. For example, at least 90% by weight over the weight of all perfumery molecules present in the fragrance may be represented by one or more perfumery molecules having water solubility at 20ºC comprised between 0.0005 g/100ml, such as 0.002g/100ml, and 1g/100ml.

Examples of perfumery molecules are one or more of:

(a) hydrocarbons, including 3-carene; alpha-pinene; beta-pinene; alpha-terpinene; gamma-terpinene; p-cymene; camphene; caryophyllene, cedrene; farnesene; limonene; longifolene; myrcene; ocimene; valencene; (E,Z)-1,3,5-undecatriene;

(b) aliphatic, alicyclic and alkyl aromatic alcohols, including hexanol; octanol; 3-octanol; 2,6-dimethylheptan-2-ol; 2,6-dimethylheptan-4-ol; 2-methylheptanol; 2-methyloctanol; (E)-3-hexenol; (E) and (Z)-3-hexenol; 1-octen-3-ol; (E,Z)-2,6-nonadienol; 3,7-dimethyl-7-methoxyoctan-2-ol; 9-decenol; 10-undecenol; 4-methyl-3-decen-5-ol; borneol; citronellol; geraniol; ethyl linalool; nerol; linalool; lavandulol; tetrahydrolinalool; tetrahydrogeraniol; dihydromyrcenol; tetrahydromyrcenol; 2,6-dimethyl-3,5-octadien-2-ol; 3,7-dimethyl-4,6-octadien-3-ol; menthol; isopulegol; alpha-terpineol; terpineol-4; menthan-8-ol; menthan-1-ol; menthan-7-ol; isoborneol; nopol; vetiverol; guaiol; benzyl alcohol; 1-phenylethyl alcohol; 2-phenylethyl alcohol; 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol; alpha-3,3-trimethylcyclohexylmethanol; 2-methyl-4-(2,2,3-trimethyl-3-cyclopent-1-yl)butanol; -2-methyl-4-(2,2,3-trimethyl-3-cyclopent-1-yl)-2-buten-1 ol; 2-ethyl-4-(2,2,3-trimethyl-3-cyclopent-1-yl)-2-buten-1-ol; 3-methyl-5-(2,2,3-trimethyl-3-cyclopent-1-yl)-pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol; 4-tert.-butylcyclohexanol; 2-tert butylcyclohexanol, 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol; (1S,2R,5S,7R,8R)-2,6,6,8 tetramethyltricyclo[5.3.1.01,5]undecan-8-ol; and the corresponding formate, acetate, propionate, isobutyrate, butyrate, isovalerate, pentanoate, hexanoate, crotonate, glycolate, 3-methyl-2-butenoate esters thereof;

(c) phenols, phenyl ethers and phenyl esters including: estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; 2-methoxy-4-propylphenol; p-cresyl phenylacetate.

(d) aliphatic, cycloaliphatic, alkyl aromatic and aromatic aldehydes, including hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-5,9-undecadienal; heptanal diethyl acetal; 1,1-dimethoxy-2,2,5-trimethyl-4-hexene; citronellyl oxyacetaldehyde; alpha-sinensal; beta-sinensal; 2,4-dimethyl-3-cyclohexenecarbaldehyde; 2-methyl-4-(2,2,6-trimethyl-cyclohexen-1-yl)-2-butenal; 4-(4-hydroxy-4-methylpentyl)-3-cyclohexenecarbaldehyde; 4-(4-methyl-3-penten-1-yl)-3-cyclohexenecarbaldehyde; geranial; neral; citronellal; 7-hydroxy-3,7-dimethyloctanal; 7-methoxy-3,7-dimethyloctanal; 2,6,10-trimethyl-9-undecenal; benzaldehyde; phenylacetaldehyde; 3-phenylpropanal; hydratropaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert.-butylphenyl)propanal; 3-(4-tert.-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; vanillin; ethyl vanillin; vanillin isobutyrate; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal; and the dimethyl, diethyl and propylene glycol acetals thereof.

(e) aliphatic, alicyclic, and alkylaromatic ketones including 2-heptanone; 2-octanone; 3-octanone; 2-nonanone; 5-methyl-3-heptanone; 2,4,4,7-tetramethyl-6-octen-3-one; menthone; isomenthone; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methanonaphthalen-8(5H-)-one; 1-(3,3-dimethylcyclohexyl)-4-penten-1-one; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl-2,6,10-trimethyl-2,5,9-cyclododecatrienyl ketone; tert.-butyl(2,4-dimethyl-3-cyclohexen-1-yl)ketone; acetophenone; 4-methylacetophenone; 4-methoxyacetophenone; 4-tert.-butyl-2,6-dimethylacetophenone; 4-phenyl-2-butanone; 4-(4-hydroxyphenyl)-2-butanone; 1-(2-naphthalenyl)ethanone; benzophenone; 1,1,2,3,3,6-hexamethyl-5-indanyl methyl ketone; 6-tert.-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone; 4-tert.-butylcyclohexanone; 2,2,5-trimethyl-5-pentylcyclopentanone; 2-heptylcyclopentanone; 2-pentylcyclopentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopentadecenone; 3-methyl-5-cyclopentadecenone; 3-methylcyclopentadecanone; 4-(1-ethoxyvinyl)-3,3,5,5-tetramethylcyclohexanone; 4-tert.-pentylcyclohexanone; 5-cyclohexadecen-1-one; 6,7-dihydro-1,1,2,3,3-pentamethyl-4(5H)-indanone; 9-cycloheptadecen-1-one; cyclopentadecanone; cyclohexadecanone;

(f) cyclic, cycloaliphatic and alkyl aromatic ethers, including cineol; cedryl methyl ether; cyclododecyl methyl ether; (ethoxymethoxy)cyclododecane; alpha-cedrene epoxide; 3a,6,6,9a-tetramethyl-dodecahydronaphtho[2,1-b]furan; 3a-ethyl-6,6,9a-trimethyldodecahydro-naphtho[2,1-b]furan; 1,5,9-trimethyl-13-oxabicyclo[10.1.0]trideca-4,8-diene; rose oxide; 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxin; 4,4a,5,9b-tetrahydro-2,4-dimethylindeno[1,2-d]-m-dioxin;

(g) aliphatic nitriles, including 2-nonenenitrile; 2-tridecenenenitrile; 2,12-tridecenenenitrile; 3,7-dimethyl-2,6-octadienenitrile; 3,7-dimethyl-6-octenenitrile;

(h) esters of aliphatic, cycloaliphatic, alkyl aromatic and aromatic carboxylic acids, including (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octynoate; methyl 2-nonynoate; allyl-2-isoamyloxyacetate; methyl-3,7-dimethyl-2,6-octadienoate; allyl 3-cyclohexylpropionate; allyl cyclohexyloxyacetate; methyl dihydrojasmonate; methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate; benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;

(i) lactones, including 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 1,15-pentadecanolide; cis and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin; and

(j) nitrogen-containing aromatic compounds, including methyl anthranilate; methyl N-methylanthranilate; Schiff bases of methyl anthranilate with 7-hydroxy-3,7-dimethyloctanal; 2-methyl-3-(4-tert.-butylphenyl)propanal or 2,4-dimethyl-3-cyclohexene-carbaldehyde; 6-isopropylquinoline; 6-isobutylquinoline; 6-sec.-butylquinoline; indole; 2-methoxy-3-isopropylpyrazine; 2-isobutyl-3-methoxypyrazine.

It is convenient that fragrances for incorporation in a perfume composition as presently disclosed be selected so that the perfume composition contains less than 25%, such as less than 15%, for example less than 5% by weight of a perfumery molecule selected from the group consisting of limonene (CAS: 5989-27-5), carvone (CAS: 99-49-0, 2244-16-8), ethyl safranate (CAS: 35044-57-6), myrcene (CAS: 123-35-3), myrcenol (CAS: 543-39-5), myrcenyl acetate (CAS: 1118-39-4), eugenol (CAS: 97-53-0), eugenyl acetate (CAS: 93-28-7), chavicol (CAs: 501-92-8), estragol (CAS: 140-67-0), anethol (CAS: 104-46-1), and mixtures thereof.

The perfume composition may also include a perfumery acceptable solvent. Solvents are conventionally used in the fragrance industry to dilute olfactively powerful ingredients and to facilitate the handling of solid ingredients by dissolving them and handling them as liquids, or simply as a diluent to reduce overall fragrance cost per unit weight. Typically, suitable solvents are water-immiscible solvents, for example solvents having water solubility of less than 10g/L. Examples of perfumery acceptable solvents are water insoluble hydrocarbon solvents (such as the Isopar® family from ExxonMobil), benzyl benzoate, isopropyl myristate, dialkyl adipates, citrate esters (such as acetyl triethyl citrate and acetyl tributyl citrate) and diethyl phthalate. If present, water miscible solvents (e.g. solvents with water solubility of more than 10g/100ml), such as propylene glycol dipropylene glycol, and butylene glycols should preferably be dosed at as low level as possible.

The perfume composition may also include benefit agents. Benefit agents are typically emulsifiable materials having synthetic or natural origin and which can survive storage to deliver a benefit through the use a product containing the microcapsules, such as household, personal care or cosmetic products. Examples of benefit agents are:

- agents which suppress or reduce malodour and its perception by adsorbing odour such as zinc ricinoleate,
- agents improving microcapsule physical-chemical properties such as sucrose octa-acetate or sucrose hexabutyrate di-acetate,
- gelling agents such as hydroxy fatty acids or the Sylvaclear™ range of materials available from Arizona Chemicals,
- agents which provide a warming or cooling effect such as cyclohexane carboxamide N-ethyl-5-methyl-2-(1-methylethyl); N 2,3-trimethyl-2-isopropylbutamide; menthyl lactate; (-)-menthoxypropane 1,2-diol,
- insect repellents such as ethylbutylacetylaminopropionate; N,N-diethyl toluamide; 1-piperidinecarboxylic acid; 2-(2-hydroxyethyl)-1-methylpropyl ester; p-menthane-3,8-diol,
- antimicrobial agents such as triclosan™ compound having CAS N° 3380-34-5, or the methyl, ethyl, propyl and butyl para hydroxy benzoate esters,
- UV absorbers such as octyl methoxycinnamate, butylmethoxydibenzoylmethane, and bis ethylhexyloxyphenolmethoxyphenyltriazine.

The present disclosure discloses a product comprising an aqueous dispersion as defined above. The product may be a non-edible consumer goods product, a household cleaner or laundry product, a personal care product or a cosmetic product.

Unless otherwise indicated, non-edible means non-intended for ingestion by humans or animals. This includes non-food products that may accidentally be swallowed during normal use. Notably, included within the definition of non-edible products are products for dental and oral care, such as toothpastes, mouth washes and lip balms which although not intended for ingestion may nevertheless accidentally enter the gastro-intestinal tract.

The formulations and ingredients of liquid household, laundry, personal care and cosmetic products in which

microcapsules of the invention may be used are well known to those skilled in the art, reference may be made to the following works:

- Formulating Detergents and Personal Care Products A guide to Product Development by L Ho Tan Tai, ISBN 1-893997-10-3 published by the AOCS Press
- Volume 67 of the Surfactant Science Series Liquid Detergents ISBN 0-8247-9391-9 (Marcel Dekker Inc),
- Harry's Cosmeticology published by CHS Press 8th Edn. 2000 ISBN 0820603724.

Personal care and cosmetic products may include products that can be applied to the skin, hair and nails either as leave on or rinse off product. Personal care and cosmetic products include powders, creams, emulsions, lotions, gels and oils for the skin (face, hands, feet etc), tinted bases (liquids and pastes) and liquid impregnated tissues; products for applying and removing make-up from the face and eyes; hair care products including hair tints and bleaches; products for waving, straightening, setting and fixing hair; shaving products including creams, foams mousses and depilatory products; sun bathing products and products for tanning without the sun; deodorant and antiperspirant products.

Advantageously a personal care or cosmetic product is selected from the group consisting of a shaving aid, a shampoo, a hair-conditioner product, a leave-on-skin-care product, a skin cleansing or washing product (such as a rinse-off skin cleansing or washing product), a moist tissue and a body spray, deodorant or antiperspirant.

Shaving aids specifically include foams, gels, creams and bars (reference can be made for example to US7,069,658, US6,944,952, US6,594,904, US6,182,365, US6,185,822, US6,298,558 and US5,113,585).

Shampoos and hair conditioners specifically include two-in-one shampoos and shampoos especially formulated for dry or greasy hair or containing additives such as antidandruff agents. Hair conditioners may be rinse off or leave on hair conditioners also included are hair tonics, bleaches colorants, setting and styling products. Reference can be made for example to US 6,162,423, US 5,968,286, US 5,935,561, US 5,932,203, US 5,837,661, US 5,776,443, US 5,756,436, US 5,661,118, US 5,618,523.

Leave-on-skin-care products comprise skin washing products, moist tissues, body sprays, deodorants and antiperspirants.

Skin washing products specifically include beauty and hygiene bar soaps, shower gels, liquid soaps, body washes, exfoliating gels and pastes (reference can be made for example to US3,697,644; US4,065,398; US4,387,040).

Moist tissues (wipes) specifically include skin cleansing wipes, baby wipes, make-up removal wipes and skin refreshing wipes (reference can be made for example to US4,775,582; WO02/07701; WO2007/069214 and WO95/16474).

Body sprays, deodorants and antiperspirants specifically include sticks, liquid roll-on applicators and pressurized sprays.

Examples of household cleaners and laundry products are:

- hard surface cleaners such as cleaners for floors, solid work surfaces, tiled surfaces, crockery by hand or machine washing and mirrors and glass,
- soft furnishing treatments such as liquid cleaners and refresher products such as odour treatment agents as exemplified by Febreze® (P&G),
- powdered laundry detergents, detergent tablets and bars, laundry detergent liquids include light duty liquids, heavy duty liquids, concentrated liquid detergents, non or low aqueous laundry liquids and more specialised cleaners for woollen or dark garments,
- fabric softeners and pre- and post-wash treatments such as tumble drier sheets, ironing waters and wash additives.

Advantageously, a laundry product is selected from the group consisting of a fabric softener, a fabric conditioner and a laundry detergent.

Household cleaners may be in the form of cream cleaners, isotropic liquid cleaners, spray cleaners and pre-moistened surface cleaning wipes (reference can be made for example to WO91/08283, EP743280, WO96/34938, WO01/23510, and WO99/28428).

Fabric softeners and conditioners specifically include both conventional diluted (e.g. 2% to 8% by weight of softener in the product) liquid active concentration softeners and concentrated (e.g. 10% to 40% by weight of softener in the product) liquid active concentration softeners as well as fabric conditioners which may contain ingredients to protect colors or garment shape and appearance (reference can be made for example to US 6,335,315, US 5,674,832, US 5,759,990, US 5,877,145, US 5,574,179).

Laundry detergents, particularly liquid laundry detergents, specifically include light duty liquid detergents and heavy duty liquid detergents which may be structured multi-phase liquids or isotropic liquids and which may be aqueous or non-aqueous liquids. These liquids may be in bottles or unit dose sachets and they may optionally contain bleaching agents or enzymes (reference can be made for example to US 5,929,022, US 5,916,862, US 5,731,278, US 5,470,507,

US 5,466,802, US 5,460,752, and US 5,458,810).

The products presently disclosed may contain water and/or surface active material, either as an emulsifier, if the product is an emulsion, or as a detergent active material if the product has some kind of cleaning function. In certain embodiments the concentration of surface active material in the product will be within the range 0.1-60% by weight; usually the level of surface active material will be 50% by weight or lower; for most products the level of surface active material will be 30% by weight or lower. On the other hand, the level of surface active material will usually be at least 0.1% by weight preferably greater than 1.0% and more preferably greater than 3.0% by weight. Certain product formulations are water sensitive (e.g. anti-perspirant, deodorant formulations, non-aqueous liquids packaged in water soluble polyvinyl alcohol films), and for these applications it may be desirable to spray dry the microcapsules to remove water, before the microcapsules are incorporated in the product formulation. For products which have a cleaning function it is likely the level of surface active material will be higher, typically greater than 10% by weight and preferably greater than 15% by weight. All percentages are expressed by weight over the weight of the product.

Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make-up application or removal, or to apply sunscreen compounds or sunless tanning agents, for personal cleansing e.g. as moist toilet tissue or baby wipes.

Examples of personal cleansing products containing detergents are: shampoos, body washes, liquid soaps. Some cleaning products may be considered leave on products even though they are used for cleansing if there is no rinsing or further cleaning action after use. Baby wipes are an example, although used for cleaning the liquid deposited on the skin is not removed by rinsing.

The non-rinsed cosmetic, toiletry and personal care compositions described herein can contain various emulsifiers which are useful for emulsifying the various components of the products. Suitable emulsifiers can include any of a wide variety of non-ionic, cationic, anionic, and zwitterionic surface active materials as disclosed in publications such as McCutcheon's, Detergents and Emulsifiers, North American Edition (1986), published by Allured Publishing Corporation and in the following patents: U.S. 5,011,681; U.S. 4,421,769; and U.S. 3,755,560.

Experimental evidence shows that the composition of certain products such as setting lotions, eau de toilettes, body spray aerosols, hair foams, which contain short hydrocarbon chain alcohols may negate the benefit brought about by the microcapsules presently disclosed. Therefore, it is preferable that the products do not contain significant amounts (e.g. more than 2.5% or more than 5%, such as more than 10%, or more than 20% or more than 50% or more than 70% by weight over the weight of the product) of short hydrocarbon chain alcohols such as aliphatic $C_1$-$C_4$ alcohols (e.g. ethanol or isopropanol). Without wishing to be bound by any theory, it is believed that short hydrocarbon chain alcohols might affect the microcapsule integrity thereby facilitating the leakage of the perfume content.

Microcapsules amount into liquid household, laundry, personal care and cosmetic products may vary depending on several aspects such as the desired microcapsule concentration, the proportion of fragrance within the microcapsule and the amount of fragrance necessary to create the olfactory effect desired. After removing all liquid components from a given product (i.e. measured as dry weight) the a plurality of microcapsules may be present from 0.01 to 10% by weight, preferably from 0.05% to 2.5% by weight, more preferably from 0.1 to 1.25% by weight over the weight of the product. The a plurality of microcapsules may be incorporated into the products by any conventional means, usually in the form of dispersion added at a suitable stage in the product manufacturing process but usually after any high shear mixing stage. If liquid at room temperature, it is preferable that the product into which the microcapsules are to be added has a viscosity greater than 20Mpas, for example greater than 100, or greater than 1,000, or even greater than 10,000Mpas, when measured at a low (e.g. 10rpm) spindle speed. Conveniently, the product shows shear thinning rheology. If necessary, viscosity can be adjusted through the addition of conventional viscosity modifying agents. Suitable agents as well as equipment and conditions to measure the viscosity of a product are discussed in Rheology Modifiers Handbook Practical Uses and Applications by M R Rosen and D Braun published by William Andrew Publishing in 2000 with ISBN 978-0-8155-1441-1.

Microcapsules may be prepared using a range of known conventional methods such as coacervation, interfacial polymerization, free radical polymerization, or polycondensation. These techniques are well-know, see e.g., US3516941, US4520142, US4528226, US4681806, US4145184; GB-A-2073132; WO99/17871; and MICROENCAPSULATION Methods and Industrial Applications Edited by Benita and Simon (Marcel Dekker, Inc. 1996) .

Advantageously, the aqueous dispersion presently disclosed may be manufactured by free radical polymerization (e.g. suspension free-radical polymerization). Accordingly, the present disclosure discloses a free radical polymerization process for the manufacture of an aqueous dispersion including a plurality of microcapsules as defined above, said process including the following steps:

a) providing an oil-in-water emulsion having an oil phase and a water phase, said emulsion being obtainable by mixing:

- ◦ solid colloidal particles having an average primary particle size comprised between 5nm and 1μm as defined above,
- ◦ a polymerization initiator,
- ◦ a perfume composition including a fragrance,
- ◦ an emulsifier, and
- ◦ a monomer blend as defined above,

b) triggering polymerization within the emulsion obtained in step a),
c) letting the polymerization propagate thereby obtaining microcapsules.

wherein the fragrance represents between 20% and 70% by weight of the weight of the emulsion.

Preferably, the fragrance represents between 20% and 45% by weight of the weight of the emulsion. Greater amounts, such as up to 70% by weight may be used to compensate for the possible dilution brought about by addition of optional process ingredients such as optional additions of monomers in the course of the process, as disclosed below.

Similarly, the overall amount of certain ingredients (e.g. colloids such as PVA) may be split and added at different stages during the process. If these ingredients are for example water soluble ingredients, the extra water brought into the reaction environment may determine a diluting effect.

The fragrance amount of 20%-45% as calculated by weight over the weight of the dispersion may conveniently be measured at any point in time throughout process step c) of letting the polymerization propagate thereby obtaining microcapsules. For example, it can be measured at the very end of step c) or towards the end of the polymerization. In effect, it might be desirable to add/remove some water to/from the final dispersion at the very end of step c) so that the concentration of the fragrance in the dispersion might appear to be lower/higher than the claimed range.

In one embodiment, the process does not include any post-polymerization concentration step to be performed on the dispersion. In effect, the dispersion presently disclosed already contains a fragrance loading which makes it suitable for direct incorporation into final products.

Steps a) to c) may be performed in the order in which they are presented.

In one aspect, the present disclosure discloses an aqueous dispersion including a microcapsule and which is obtainable by a free radical polymerization process as defined above.

Polymerization may be conventional radical polymerization or living radical polymerization. Such radical polymerization processes are known to persons skilled in the art and are further described e.g. in Moad, Graeme; Solomon, David H.; The Chemistry of Radical Polymerization, 2nd ed.; Elsevier, 2006. A discussion of living radical polymerization, can be found for example in Braunecker, Wade A.; Matyjaszewski, Krzysztof; "Controlled/Living Radical Polymerization: Features, Developments, and Perspectives"; Progress in Polymer Science 2007, Volume 32, Issue 1, Pages 93-146.

The monomers of the blend are as defined above. They are weighed and mixed so as to obtain a monomer blend as defined above. Then, this blend is used in the preparation of the oil-in-water emulsion.

An oil-in-water emulsion (step a)) may be prepared by mixing and dissolving the oil soluble ingredients into a homogeneous solution while separately mixing and dissolving the water soluble ingredients into a homogenous solution. Solid colloidal particles are typically admixed to the water solution. An emulsion may be obtained by mixing e.g. with a high shear mixer and for sufficient time the two solutions to create a stable emulsion of a desired particle size. At the same time the emulsion may be purged with nitrogen or other inert gas. Once the air has been removed, polymerization may be heat induced (step b)) by elevating the temperature. The exact temperature and rate of temperature increase is determined by the initiator or combination of initiators to be used. Typically polymerization temperatures are between 40°C to 90°C. The rate of polymerization can be controlled in a known manner by appropriate choice of the temperature and amount of polymerization initiator for the particular monomers and initiator in an experiment. Once the polymerization temperature has been reached, polymerization continues (step c)) for a further period, for example 2 to 6 hours, in order to complete the reaction of the monomers.

Additional initiator can be added later in the polymerization to reduce the level of residual monomers. Monomers may be added during the course of the reaction to control dosage. Salts may be added e.g. to buffer the pH.

The emulsion includes a polymerization initiator. Radicals can be generated by thermal decomposition of compounds such as peroxy and azo compounds, or by photolysis with UV radiation or by redox reactions. Suitable Initiators may be soluble in the oil phase and/or the aqueous phase of the emulsion. For example, an initiator may be:

- a thermal polymerization initiator, and/or
- a photopolymerization initiator, and/or
- a redox initiator including a radical-generating reductant/oxidant pair.

Thermal polymerization initiators may be present in an amount comprised between 0.1% and 5% by weight over the combined weight of compounds (I) and (II) in the blend.

Examples of thermal polymerization initiator are:

dilauroyl peroxide,
benzoyl peroxide,
$\alpha,\alpha'$-azoisobutyronitrile,
2,2'-azobis(2.4-dimethyl valeronitrile),
dimethyl 2,2'-azobis(2-methylpropionate),
1,1'-azo-bis-1-Cyclohexanenitrile,
di-tert-butyl peroxide (CAS: 75-91-2),
potassium persulphate,
ammonium persulfate,
4,4'-azobis(4-cyanovaleric acid),
2,2'-azobis[2-(2-imidazolin-2-yl)propane]dihydrochloride,
2,2'-azobis(2-methylpropionamidine)dihydrochloride,
2,2'-azobis[2-(2-imidazolin-2-yl)propane],
2,2'-azobis[2-methyl-N-(2-hydroxyethyl)propionamide], and
mixtures thereof.

Photopolymerization initiators may be present in an amount comprised between 0.5% and 5% by weight over the combined weight of compounds (I) and (II) in the blend.

Examples of photopolymerization initiator are:

alpha hydroxyl ketones,
alpha amino ketones,
alpha and beta naphthyl carbonyl compounds,
benzoin ethers such as benzoin methyl ethers,
benzophenone,
acetophenone,
benzaldehyde,
xanthone,
9,10-anthraquinone,
1-hydroxy-cyclohexyl-phenyl-ketone (Irgacure™ 184), and
mixtures thereof.

A redox initiator includes a radical-generating reductant/oxidant pair. In the pair

- the oxidant may be present in an amount comprised between 0.01% and 3.0%, such as between 0.02% and 1.0%, or between 0.05% and 0.5% by weight over the combined weight of compounds (I) and (II) in the blend, and/or
- the reductant may be present in an amount comprised between 0.01% and 3.0%, such as between 0.01% and 0.5%, or between 0.025% and 0.25% by weight over the combined weight of compounds (I) and (II) in the blend.

Examples of oxidant for the redox pair are:

- salts of peroxodisulfuric acid such as sodium monopersulfate, sodium persulfate, potassium persulphate, ammonium persulfate,
- cumene hydroperoxide,
- tert-butyl hydroperoxide,
- di-tert-amyl peroxide,
- tert-butyl peroxybenzoate,
- t-amyl hydroperoxide,
- hydrogen peroxide, and
- mixtures thereof

Examples of reductant for the redox pair are:

- sodium sulphite,
- sodium metabisulphite,
- sodium formaldehyde sulphoxylate,

- ascorbic acid,
- sodium dithionite, and
- mixtures thereof.

The emulsion includes an emulsifier. The emulsifier includes a protective colloid and may further include a surfactant. Protective colloids and surfactants are conventionally used in emulsion polymerization and in suspension polymerization to stabilize oil-in-water emulsions created by mechanical agitation while the polymerization occurs.
A suitable protective colloid has an average molecular weight comprised between 500 and 1.000.000 g/mol, for example between 1.000 and 500.000 g/mol.
Examples of protective colloid are:

- cellulose derivatives such as hydroxyethylcellulose, carboxymethylcellulose and methylcellulose,
- polyvinylpyrrolidone,
- copolymers of N-vinylpyrrolidone,
- polyvinyl alcohols obtainable by full to partial hydrolyses of polyvinyl acetates,
- polyacrylic and/or polymethacrylic acid,
- copolymers of acrylic acid and methacrylic acid,
- ionic colloids such as sulphonic-acid-group-containing water-soluble polymers (e.g. 2-acrylamido-2-alkylsulphonic acids and styrene sulphonic acids), and
- mixtures thereof.

Advantageously, the protective colloid is a water-soluble protective colloid. Preferably, this means that the colloid has solubility in water of at least 5 g/L at 20ºC.
Advantageously, the protective colloid includes at least polyvinyl alcohol (PVA), such as a PVA obtainable by full to partial hydrolyses of polyvinyl acetates.
The protective colloid may be present in an amount comprised between 0.1% and 10% by weight over the weight of the water phase of the oil-in-water emulsion.
Step b) entails inducing decomposition of polymerization initiator. Polymerization may be initiated either in the oil phase (suspension polymerization) or the water phase (emulsion polymerization) of the emulsion depending on the choice of the initiator(s). It is also possible to initiate polymerization in the two phases separately by appropriate choice of initiator and conditions. Step b) may comprise:

- subjecting the oil-in-water emulsion to heat, and/or
- subjecting the oil-in-water emulsion to UV light, and/or
- triggering a redox reaction within the oil-in-water emulsion.

The microcapsules of the invention may also comprise on their surface (e.g. surface grafted) deposition aids, i.e. aids aiming to optimize the deposition of microcapsule on the intended substrate (examples of substrates are hair, skin and fabrics such as cotton). Examples and use of deposition aids on microcapsules are for example disclosed in EP21558474, EP1572767, EP2188364 and EP1019478.
The deposition aid may be present in an amount comprised between 0.1% and 10% by weight over the dry weight of a microcapsule.
The deposition aid may be a polymeric deposition aid. Examples may be synthetic or natural polymers or combinations thereof (e.g. through partial chemical modification of natural polymers).
The deposition aid may be a peptide, a protein, or a chemical derivative thereof, providing for a binding to the intended substrates. For example cellulases bind to cotton while proteases bind to wool, silk or hair.
The deposition aid may be a polysaccharide or a chemical derivative thereof. The polysaccharide preferably has a [beta]-1,4-linked backbone. Examples of polysaccharide are cellulose, a cellulose derivative, or another [beta]-1,4-linked polysaccharide binding to cellulose, such as polymannan, polyglucan, polyglucomannan, polyxyloglucan and polygalactomannan or mixtures thereof. For example, the polysaccharide is selected from the group consisting of polyxyloglucan and polygalactomannan. Highly preferred polysaccharides are selected from locust bean gum, tamarind gum, xyloglucan, non-ionic guar gum, cationic starch and mixtures thereof. For example, the deposition aid is locust bean gum, or chemical derivatives thereof.
In one embodiment, the process presently disclosed may include a step d) to be performed after step c) and including binding a deposition aid to the microcapsules in the plurality of microcapsules. The deposition aid may be adsorbed to the microcapsule shell or physically and/or chemically bonded to the microcapsule shell. Adsorption (i.e. physical binding) of the deposition aid to the already-formed microcapsule shell may rely on hydrogen bonding, Van Der Waals or electrostatic attraction between the deposition aid and the microcapsule. The deposition aid is thus external

to the microparticle and is not, to any significant extent, within the shell and/or within the microcapsule core.

Alternatively, a deposition aid may be part of the emulsion provided in step a). In this case, the deposition aid will be integral part of the microcapsule shell. This situation is known as "entanglement". By entanglement as used herein is meant that the deposition aid is partially buried within the interior of the microcapsule. This is obtained by adding the deposition aid to the emulsion e.g. before the polymerization is triggered. By letting the polymerization propagate, part of the deposition aid remains entrapped and bound in the extending polymer that will form the microcapsule shell whilst the remainder is free to extend into the aqueous phase of the emulsion. In this manner, the deposition aid is only partially exposed at the microcapsule surface.

Further embodiments and advantages of the present invention will become apparent to a skilled reader in light of the examples provided below.

Two alternatives for the General manufacturing process are disclosed. Alternative 1 is followed for monomer blends that do not comprise monomers with hydroxyl groups or monomers that are not solubilized in the fragrance. Alternative 2 is followed for monomer blends that comprise monomers with hydroxyl groups and/or monomers that are not solubilized in the fragrance. By "solubilized in the fragrance", it is meant that the amount of monomer considered is fully solubilized in the fragrance, forming a monophasic, homogeneous and transparent phase.

General manufacturing process (alternative 1)

A 10 % poly(vinyl alcohol) aqueous solution was prepared in advance by dissolving poly(vinyl alcohol), hydrolyzed to 87-89 %, $M_w$=85000-124000 g/mol in water. An oil phase was prepared by first mixing the fragrance and the monomers to obtain a monophasic, homogeneous and transparent phase. The polymerization initiator was then added and the mixture was stirred until complete dissolution of the polymerization initiator. A dispersion of silica in water was prepared separately by stirring during 5 min the Aerosil R816 silica and the water with a pH between 6.5 and 8.5. The water dispersion contained sodium bicarbonate 100mg/L (to approximately have a pH in the range of 6.5 to 8.5. The oil phase and the dispersion of silica in water were stirred together at 7000 rpm for 2 min using a high-shear mixer (Ystral X 10/20 E3-1050 W equipped with a Dispermix head of diameter 40/54 mm). The mean particle and the span number of the resultant emulsion were determined according to the capsule particle size measurement method disclosed below. The emulsion was placed into a batch reactor equipped with a condenser, a thermometer, a nitrogen inlet and an anchor stirrer. A known amount of 10 % poly(vinyl alcohol) aqueous solution was added to get a total weight concentration of poly(vinyl alcohol) in the water phase of 2 % and the mixture was stirred during 10 min. During all the process, the mixture was stirred at 250 rpm and nitrogen was bubbled through the mixture to remove oxygen. The temperature is first fixed at a temperature T1 during 30 min and the temperature is then increased to the temperature T2 within one hour. The mixture is kept at this temperature T2 during 3 hours. Finally, the resultant microcapsule dispersion was cooled to room temperature within 1 hour. The mean particle and the span number of the resultant microcapsule dispersion were determined according to the capsule particle size measurement method disclosed below.

General manufacturing process (alternative 2)

A 10 % poly(vinyl alcohol) aqueous solution was prepared in advance by dissolving poly(vinyl alcohol), hydrolyzed to 87-89 %, $M_w$=85000-124000 g/mol in water. An oil phase was prepared by mixing the fragrance and the monomers which are soluble in the fragrance except the monomer with hydroxyl groups. A monophasic, homogeneous and transparent phase was obtained. The polymerization initiator was then added and the mixture was stirred until complete dissolution of the polymerization initiator. This mixture was stirred until complete dissolution of the polymerization initiator. The water dispersion contained sodium bicarbonate 100mg/L (to approximately have a pH in the range of 6.5 to 8.5. In water were introduced in the following order: the monomers with hydroxyl groups and/or the neutral monomers that are not soluble in the fragrance, a 1 % solution of 3-(methacryloylamino)propyl]trimethylammonium chloride (MAPTAC, CAS 51410-72-1) in water and the Aerosil® 200 silica. The weight of the 1 % solution of MAPTAC in water represents between 0.5 % and 100 % of the weight of silica. The dispersion was stirred during 30 min. The water dispersion pH range was within a pH of 6.5 to 8.5. The oil phase and the dispersion of silica in water were stirred together at 7000 rpm for 2 min using a high-shear mixer (Ystral X 10/20 E3-1050 W equipped with a Dispermix head of diameter 40/54 mm). The mean particle and the span number of the resultant emulsion were determined according to the capsule particle size measurement method disclosed below. The emulsion was placed into a batch reactor equipped with a condenser, a thermometer, a nitrogen inlet and an anchor stirrer. A known amount of 10 % poly(vinyl alcohol) aqueous solution was added to get a total weight concentration of poly(vinyl alcohol) in the water phase of 2.6 % and the mixture was stirred during 10 min. If present, ionized monomers (which are not soluble in the fragrance) may be added at this stage. During all the process, the mixture was stirred at 250 rpm and nitrogen was bubbled through the mixture to remove oxygen. The temperature is first fixed at a temperature T1 during 30 min and the temperature is then increased to the temperature T2 within one hour. The mixture is kept at this temperature T2 during 3 hours. Finally, the

resultant microcapsule dispersion was cooled to room temperature within 1 hour. The mean particle and the span number of the resultant microcapsule dispersion were determined according to the capsule particle size measurement method disclosed below.

Capsule particle size measurement

Median volume diameter and span were measured with a laser diffraction/scattering particle size distribution analyzer (trade name: LA-950V2, manufactured by Horiba, Ltd.). The dispersant was 18 MΩ water. Several droplets of the emulsion or the capsule dispersion were poured into the flow cell unit until an acceptable level of laser light obscuration was achieved and triplicate measurements were then immediately performed. For the calculation of the particle size measurement, the refractive indexes were set at 1.33 (for the water dispersant), 1.47 (for the fragrances and the poly(methacrylate) capsules). The median capsule diameter was measured as a particle size of 50% frequency (median size) on a volumetric basis.

The span value is an indication of microcapsule size statistical dispersion. It is presently calculated according to the following formula:

$$Span = \frac{D(v;0.9) - D(v;0.1)}{D(v;0.5)}$$

in which D(v; 0.9) is the particle size for 90% of the microcapsules by volume, D(v; 0.1) is the particle size for 10% of the microcapsules by volume and D(v; 0.5) is the median volume microcapsule size as previously defined.

The span ratio value is the ratio between the Span value of the aqueous dispersion and the Span value of the initial (oil-in-water) emulsion. It is presently calculated according to the following formula:

$$Span\ ratio = \frac{Span\ Capsule}{Span\ Emulsion}$$

wherein Span Capsule is the span as defined above of the aqueous microcapsule dispersion and the Span Emulsion is the span as defined above of the initial emulsion.

Since the particle size may be larger than 10μm the analysis of the results by the Fraunhofer approximation (opaque particles, geometrical optic rules) is also relevant and lead valid size determination. In this case the refractive index is not necessary.

| *Composition of fragrance no. 1* | *(% by weight):* |
|---|---|
| Isobornyl acetate (CAS N° 125-12-2) : | 25 |
| Camphor gum powder synthetic (CAS N° 464-49-3) : | 15 |
| Lilial (CAS N° 80-54-6) : | 15 |
| Eucalyptol (CAS N° 470-82-6) : | 8 |
| Ethyl-2-methylpentanoate (CAS N° 39255-32-8) : | 6 |
| Cedrol (CAS N° 77-53-2) : | 6 |
| Allyl heptoate (CAS N° 142-19-8) : | 5 |
| Styrallyl acetate (CAS N° 93-92-5) : | 5 |
| 2-Methylundecanal (CAS N° 110-41-8) : | 5 |
| Verdox (CAS : 88-41-5) | 5 |
| Coumarin (CAS N° 91-64-5) : | 3 |
| Delta damascone (CAS N° 57378-68-4) : | 2 |

**Example 1:** Synthesis of the capsules according to the invention

The general manufacturing process was followed for to prepare microcapsule samples 1 and 2. A 10 % poly(vinyl alcohol) aqueous solution was prepared in advance by dissolving poly(vinyl alcohol), hydrolyzed to 87-89 %, $M_w$=85000-124000 g/mol in water. An oil phase was prepared by mixing 1.23 g of benzoyl peroxide, 75% in water; 21.8

g of methacrylic acid; 8.7 g of methyl methacrylate; 24.0 g of 1,4-butane diol dimethacrylate; 150 g of fragrance no. 1. A monophasic, homogeneous and transparent phase was obtained. The polymerization initiator was then added and the mixture was stirred until complete dissolution of the polymerization initiator. A dispersion of silica in water was prepared separately by stirring during 5 min 1.20 g of Aerosil® R816 silica and water with a pH between 6.5 and 8.5. The water dispersion pH range was within a pH of 6.5 to 8.5. The oil phase and the dispersion of silica in water were stirred together at 7000 rpm for 2 min using a high-shear mixer (Ystral X 10/20 E3-1050 W equipped with a Dispermix head of diameter 40/54 mm). The mean particle and the span number of the resultant emulsion were determined according to the capsule particle size measurement method disclosed below. 360 g of the emulsion were placed into a 500 mL-batch reactor equipped with a condenser, a thermometer, a nitrogen inlet and an anchor stirrer. A known amount of 10 % poly(vinyl alcohol) aqueous solution was added to get a total weight concentration of poly(vinyl alcohol) in the water phase of 2 % and the mixture was stirred during 10 min. During all the process, the mixture was stirred at 250 rpm and nitrogen was bubbled through the mixture to remove oxygen. The temperature is first fixed at 20 °C during 30 min and the temperature is then increased to 80 °C within one hour. The mixture is kept at this temperature of 80 °C during 3 hours. Finally, the resultant microcapsule dispersion was cooled to room temperature within 1 hour. The mean particle and the span number of the resultant microcapsule dispersion were determined according to the capsule particle size measurement method disclosed below.

| Sample | Weight of water in the dispersion of silica Weight of 10 % poly(vinyl alcohol) aqueous solution (g) | Concentration of fragrance in the final capsule dispersion (%) | Median volume diameter of the capsule dispersion (μm; D(v, 0.5)); Span value |
|---|---|---|---|
| 1 | 200<br>43.1 | 32.9 | 36.5<br>0.61 |
| 2 | 165.5<br>39.1 | 36.3 | 39.3<br>0.75 |

**Example 2:** comparative examples (samples 3 and 4)

A 10 % poly(vinyl alcohol) aqueous solution was prepared in advance by dissolving poly(vinyl alcohol), hydrolyzed to 87-89 %, $M_w$=85000-124000 g/mol in water. An aqueous phase was prepared by mixing known amounts of 10 % poly(vinyl alcohol) aqueous solution and water with a pH range was within 6.5 to 8.5. An oil phase was prepared by mixing 1.23 g of benzoyl peroxide, 75 % in water; 21.8 g of methacrylic acid; 8.7 g of methyl methacrylate; 24.0 g of 1,4-butane diol dimethacrylate; 150 g of fragrance no. 1. A monophasic, homogeneous and transparent phase was obtained. The polymerization initiator was then added and the mixture was stirred until complete dissolution of the polymerization initiator. The aqueous phase and the oil phase were placed into a 500 mL-batch reactor equipped with a condenser, a thermometer, a nitrogen inlet and a deflocculating blade (diameter 4 cm). During all the process, the mixture was stirred at 900 rpm and nitrogen was bubbled through the mixture to remove oxygen. The temperature is first fixed at 20 °C during 30 min and the temperature is then increased to 80 °C within one hour. The mixture is kept at this temperature of 80 °C during 3 hours. Finally, the resultant microcapsule dispersion was cooled to room temperature within 1 hour. The mean particle and the span number of the resultant microcapsule dispersion were determined according to the capsule particle size measurement method disclosed below.

| Sample | Weight of water in the dispersion of silica Weight of 10 % poly(vinyl alcohol) aqueous solution (g) | Concentration of fragrance in the final capsule dispersion (%) | Median volume diameter of the capsule dispersion (μm; D(v, 0.5)); Span value |
|---|---|---|---|
| 3 | 200<br>49.1 | 33.0 | 37.6<br>1.01 |
| 4 | 160<br>40.0 | 37.0 | 42.6<br>1.20 |

**Example 3** - **Viscosity measurements**

The viscosity measurement of the capsules dispersions were performed at 20 °C by using a Brookfield RVT Viscometer. Depending on the viscosity of the capsules dispersions, the measurements were performed with adapted rotational spindle and speeds.

| Sample | Viscosity at 20 °C (cps) | Spindle and spindle speed |
|---|---|---|
| 1 | 120 | Spindle 1, 10 rpm |
| 2 | 1500 | Spindle 2, 10 rpm |
| 3 | 6200 | Spindle 4, 10 rpm |
| 4 | Paste (too viscous to be measured) | / |

These results show that the viscosities of the samples of the present invention are much lower to the viscosities of the comparative examples with equivalent fragrance loading. Sample 4 is no longer fluid (or mobile) and is thus not easy to handle.

## Claims

**1.** An aqueous dispersion including a plurality of microcapsules, each microcapsule comprising a perfume composition enclosed within a polymeric shell, wherein

- the perfume composition includes a fragrance,
- the shell includes solid colloidal particles having an average primary particle size comprised between 5nm and 1 μm,
- the shell further includes in polymerized form a monomer blend including:

i) between 30% and 80% by weight over the combined weight of compounds (I) to (II) in the blend of a compound (I) which is a monoethylenically unsatured monomer and/or dimethyldiallyl ammonium chloride (DMDAAC),
ii) between 20% and 70% by weight over the combined weight of compounds (I) to (II) in the blend of a compound (II) which is a polyethylenically unsatured monomer, wherein compound (II) is a C2-C24 alkyl di- or polyester of (meth)acrylic acid and:

A1 contains two or more (meth)acrylate ester groups per monomer, and
B1. has a molecular weight which, once divided by the number of (meth)acrylate ester groups, gives a value of more than 85 g/mol and lower than 135 g/mol,

wherein the combined amounts of compounds (I) and (II) make up 100% by weight of the blend, and
wherein the fragrance represents between 20% and 45% by weight over the weight of the dispersion.

**2.** The aqueous dispersion according to claim 1, wherein the fragrance represents between 30% and 45% by weight over the weight of the dispersion.

**3.** The aqueous dispersion according to any one of claims 1 to 2, wherein compound (I) is selected from (meth)acrylate monomers which are polymerizable through free-radical polymerization.

**4.** The aqueous dispersion according to any one of claims 1 to 3, wherein compound (I) is selected the group consisting of methacrylic acid, methyl methacrylate, ethyl methacrylate, isopropyl methacrylate, 2-hydroxyethyl methacrylate, 2-hydroxypropyl methacrylate, 3-hydroxypropyl methacrylate and mixtures thereof.

**5.** The aqueous dispersion according to any one of claims 1 to 2, wherein compound (I) is a combination of:

ia) between 50% and 100% by weight over the weight of the combination of a neutral monomethacrylate monomer (Ia) having a solubility in water at pH 7 and 20ºC equal to, or more of 2g/100ml,
ib) between 0% and 50% by weight over the weight of the combination of another neutral monoethylenically unsatured monomer (Ib), and
ic) between 0% and 15% by weight over the weight of the combination of a ionized or ionizable monoethylenically unsatured monomer (Ic).

6. The aqueous dispersion according to claim 5, wherein the neutral monomethacrylate monomer (Ia) is selected from the group consisting of 2-hydroxyethyl methacrylate, 2-hydroxypropyl methacrylate, 3-hydroxypropyl methacrylate, glycidyl methacrylate, poly(ethylene glycol) methyl ether methacrylate and mixtures thereof.

7. The aqueous dispersion according to any one of claims 1 to 6, wherein compound (II) is a di- or polyester resulting from the esterification of (meth)acrylic acid with a linear or branched polyhydric C2-C24 alcohol and/or C2-C24 polyethylene glycols.

8. The aqueous dispersion according to claim 7, wherein compound (II) comprises one or more of 1,4-butylene glycol dimethacrylate, ethylene glycol dimethacrylate and 1,3-propylene glycol dimethacrylate.

9. A product comprising the aqueous dispersion as defined in any one of claims 1 to 8.

10. A process for the manufacture of an aqueous dispersion as defined in any one or more of claims 1 to 8, said process comprising the following steps:

a) providing an oil-in-water emulsion having an oil phase and a water phase, said emulsion being obtainable by mixing:

- solid colloidal particles having an average primary particle size comprised between 5nm and 1$\mu$m,
- a polymerization initiator,
- a perfume composition including a fragrance,
- an emulsifier, and
- a monomer blend as defined in any one of claims 1 to 8,

b) triggering polymerization within the emulsion obtained in step a),
c) letting the polymerization propagate thereby obtaining microcapsules;

wherein the fragrance represents between 20% and 70% by weight of the weight of the emulsion.

**Patentansprüche**

1. Wässrige Dispersion, welche mehrere Mikrokapseln beinhaltet, wobei jede Mikrokapsel eine Parfümzusammensetzung umfasst, die innerhalb einer Polymerhülle eingeschlossen ist, wobei

- die Parfümzusammensetzung einen Duftstoff beinhaltet,
- die Hülle feste kolloidale Teilchen mit einer durchschnittlichen Primärteilchengröße zwischen 5 nm und 1 $\mu$m beinhaltet,
- die Hülle ferner in polymerisierter Form eine Monomermischung beinhaltet, welche beinhaltet:

i) zwischen 30 Gew.-% und 80 Gew.-% über das kombinierte Gewicht der Verbindungen (I) bis (II) in der Mischung einer Verbindung (I), welche ein monoethylenisch ungesättigtes Monomer und/oder Dimethyldiallylammoniumchlorid (DMDAAC) ist,
ii) zwischen 20 Gew.-% und 70 Gew.-% über das kombinierte Gewicht der Verbindungen (I) bis (II) in der Mischung einer Verbindung (II), welche ein polyethylenisch ungesättigtes Monomer ist, wobei die Verbindung (II) ein C2-C24-Alkyl-Di- oder -Polyester von (Meth)acrylsäure ist und:

A1 zwei oder mehr (Meth)acrylatestergruppen pro Monomer enthält, und
B1. ein Molekulargewicht aufweist, welches, sobald es durch die Anzahl der (Meth)acrylatestergruppen geteilt wird, einen Wert von mehr als 85 g/mol und weniger als 135 g/mol ergibt,

wobei die kombinierten Mengen der Verbindungen (I) und (II) 100 Gew.-% der Mischung bilden und
wobei der Duftstoff zwischen 20 Gew.-% und 45 Gew.-% über das Gewicht der Dispersion darstellt.

2. Wässrige Dispersion nach Anspruch 1, wobei der Duftstoff zwischen 30 Gew.-% und 45 Gew.-% über das Gewicht der Dispersion darstellt.

3. Wässrige Dispersion nach einem der Ansprüche 1 bis 2, wobei die Verbindung (I) aus (Meth)acrylatmonomeren ausgewählt ist, die durch eine radikalische Polymerisation polymerisierbar sind.

4. Wässrige Dispersion nach einem der Ansprüche 1 bis 3, wobei die Verbindung (I) aus der Gruppe ausgewählt ist, bestehend aus Methacrylsäure, Methylmethacrylat, Ethylmethacrylat, Isopropylmethacrylat, 2-Hydroxyethylmethacrylat, 2-Hydroxypropylmethacrylat, 3-Hydroxypropylmethacrylat und Gemischen davon.

5. Wässrige Dispersion nach einem der Ansprüche 1 bis 2, wobei die Verbindung (I) eine Kombination ist von:

ia) zwischen 50 Gew.-% und 100 Gew.-% über das Gewicht der Kombination eines neutralen Monomethacrylatmonomers (Ia), das bei einem pH-Wert von 7 und 20 °C eine Wasserlöslichkeit von gleich oder mehr als 2 g/100 ml aufweist,
ib) zwischen 0 Gew.-% und 50 Gew.-% über das Gewicht der Kombination eines anderen neutralen monoethylenisch ungesättigten Monomers (Ib), und
ic) zwischen 0 Gew.-% und 15 Gew.-% über das Gewicht der Kombination eines ionisierten oder ionisierbaren monoethylenisch ungesättigten Monomers (Ic).

6. Wässrige Dispersion nach Anspruch 5, wobei das neutrale Monomethacrylatmonomer (Ia) aus der Gruppe ausgewählt ist, bestehend aus 2-Hydroxyethylmethacrylat, 2-Hydroxypropylmethacrylat, 3-Hydroxypropylmethacrylat, Glycidylmethacrylat, Poly(ethylenglykol)methylethermethacrylat und Gemischen davon.

7. Wässrige Dispersion nach einem der Ansprüche 1 bis 6, wobei die Verbindung (II) ein Di- oder Polyester ist, das sich aus der Veresterung von (Meth)acrylsäure mit einem linearen oder verzweigten mehrwertigen C2-C24-Alkohol und/oder C2-C24-Polyethylenglykolen ergibt.

8. Wässrige Dispersion nach Anspruch 7, wobei die Verbindung (II) eines oder mehrere von 1,4-Butylenglykoldimethacrylat, Ethylenglykoldimethacrylat und 1,3-Propylenglykoldimethacrylat umfasst.

9. Produkt, umfassend die wässrige Dispersion wie in einem der Ansprüche 1 bis 8 definiert.

10. Prozess für die Herstellung einer wässrigen Dispersion wie in einem oder mehreren der Ansprüche 1 bis 8 definiert, wobei der Prozess die folgenden Schritte umfasst:

a) Bereitstellen einer Öl-in-Wasser-Emulsion, die eine Ölphase und eine Wasserphase aufweist, wobei die Emulsion erhalten werden kann durch das Mischen:

- fester kolloidaler Teilchen mit einer durchschnittlichen Primärteilchengröße zwischen 5 nm und 1 μm,
- eines Polymerisationsinitiators,
- einer Parfümzusammensetzung, welche einen Duftstoff beinhaltet,
- eines Emulgators und
- einer Monomermischung wie in einem der Ansprüche 1 bis 8 definiert,

b) Auslösen der Polymerisation in der in Schritt a) erhaltenen Emulsion,
c) Zulassen der Ausbreitung der Polymerisation, wodurch Mikrokapseln erhalten werden,

wobei der Duftstoff zwischen 20 Gew.-% und 70 Gew.-% des Gewichts der Emulsion darstellt.

## Revendications

1. Dispersion aqueuse incluant une pluralité de microcapsules, chaque microcapsule comprenant une composition de parfum incluse dans une enveloppe polymérique, où :

- la composition de parfum inclut un parfum,
- l'enveloppe inclut des particules colloïdales solides ayant une taille moyenne de particules primaires comprise entre 5nm et 1 μm,
- l'enveloppe inclut en outre sous forme polymérisée un mélange de monomères incluant :

i) entre 30% et 80% en masse, par rapport à la masse combinée des composés (I) et (II) dans le mélange, d'un composé (I) qui est un monomère insaturé monoéthylénique et/ou du chlorure de diméthyldiallyle ammonium (DMDAAC),
ii) entre 20% et 70% en masse, par rapport à la masse combinée des composés (I) et (II) dans le mélange, d'un composé (II) qui est un monomère insaturé polyéthylénique,
où le composé (II) est un di- ou poly(méth)acrylate d'alkyle en $C_2$-$C_{24}$ et :

A1 contient au moins deux groupes (méth)acrylate par monomère, et
B1. a une masse moléculaire qui, une fois divisée par le nombre de groupes (méth)acrylate, a une valeur supérieure à 85 g/mol et inférieure à 135 g/mol,

où la quantité combinée des composés (I) and (II) représente 100% en masse du mélange, et
où le parfum représente entre 20% et 45% en masse de la masse de la dispersion.

**2.** Dispersion aqueuse selon la revendication 1, dans laquelle le parfum représente entre 30% et 45% en masse de la masse de la dispersion.

**3.** Dispersion aqueuse selon l'une quelconque des revendications 1 à 2, dans laquelle le composé (I) est choisi parmi les monomères (méth)acrylate qui sont polymérisables par polymérisation radicalaire.

**4.** Dispersion aqueuse selon l'une quelconque des revendications 1 à 3, dans laquelle le composé (I) est choisi dans le groupe constitué de : acide méthacrylique, méthacrylate de méthyle, méthacrylate d'éthyle, méthacrylate d'isopropyle, méthacrylate de 2-hydroxyéthyle, méthacrylate de 2-hydroxypropyle, méthacrylate de 3-hydroxypropyle et leurs mélanges.

**5.** Dispersion aqueuse selon l'une quelconque des revendications 1 à 2, dans laquelle le composé (I) est une combinaison de :

ia) entre 50% et 100% en masse, par rapport à la masse de la combinaison, d'un monomère neutre monométhacrylate (Ia) ayant une solubilité dans l'eau à pH 7 et 20ºC supérieure ou égale à 2g/100ml,
ib) entre 0% et 50% en masse, par rapport à la masse de la combinaison, d'un autre monomère neutre monoéthylénique insaturé (Ib), et
ic) entre 0% et 15% en masse, par rapport à la masse de la combinaison, d'un monomère monoéthylénique insaturé ionisé ou ionisable (Ic).

**6.** Dispersion aqueuse selon la revendication 1, dans laquelle le monomère neutre monométhacrylate (Ia) est choisi dans le groupe constitué de : méthacrylate de 2-hydroxyethyle, méthacrylate de 2-hydroxypropyle, méthacrylate de 3-hydroxypropyle, méhacrylate de glycidyle, poly(éthylène glycol) méthacrylate de méthyl éther et leurs mélanges.

**7.** Dispersion aqueuse selon l'une quelconque des revendications 1 à 6, dans laquelle le composé (II) est un di- ou polyester résultant de l'estérification de l'acide (méth)acrylique avec un alcool polyhydrique linéaire ou ramifié en C2-C24 et/ou des polyéthylène glycols en C2-C24.

**8.** Dispersion aqueuse selon la revendication 7, dans laquelle le composé (II) comprend un ou plusieurs des composés: diméthacrylate de 1,4-butylène glycol, diméthacrylate d'éthylène glycol et diméthacrylate de 1,3-propylène glycol.

**9.** Produit comprenant la dispersion aqueuse telle que définie dans l'une quelconque des revendications 1 à 8.

**10.** Procédé de fabrication d'une dispersion aqueuse telle que définie dans l'une quelconque ou plusieurs des revendications 1 à 8, ledit procédé comprenant les étapes suivantes :

a) fourniture d'une émulsion huile-dans-eau ayant une phase huileuse et une phase aqueuse, ladite émulsion étant susceptible d'être obtenue en mélangeant :

- des particules colloïdales solides ayant une taille moyenne de particules primaires comprise entre 5nm et 1$\mu$m ,
- un initiateur de polymérisation,
- une composition de parfum incluant un parfum,

- un émulsifiant, et
- un mélange tel que défini dans au moins l'une des revendications 1 à 8 ;

b) déclenchement de la polymérisation au sein de l'émulsion obtenue à l'étape a) ;
c) laisser la polymérisation se propager pour obtenir ainsi des microcapsules ;

où le parfum représente entre 20% et 70% en masse de la masse de l'émulsion.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010145993 A
- US 7069658 B
- US 6944952 B
- US 6594904 B
- US 6182365 B
- US 6185822 B
- US 6298558 B
- US 5113585 A
- US 6162423 A
- US 5968286 A
- US 5935561 A
- US 5932203 A
- US 5837661 A
- US 5776443 A
- US 5756436 A
- US 5661118 A
- US 5618523 A
- US 3697644 A
- US 4065398 A
- US 4387040 A
- US 4775582 A
- WO 0207701 A
- WO 2007069214 A
- WO 9516474 A
- WO 9108283 A
- EP 743280 A
- WO 9634938 A
- WO 0123510 A
- WO 9928428 A
- US 6335315 B
- US 5674832 A
- US 5759990 A
- US 5877145 A
- US 5574179 A
- US 5929022 A
- US 5916862 A
- US 5731278 A
- US 5470507 A
- US 5466802 A
- US 5460752 A
- US 5458810 A
- US 5011681 A
- US 4421769 A
- US 3755560 A
- US 3516941 A
- US 4520142 A
- US 4528226 A
- US 4681806 A
- US 4145184 A
- GB 2073132 A
- WO 9917871 A
- EP 21558474 A
- EP 1572767 A
- EP 2188364 A
- EP 1019478 A


### Non-patent literature cited in the description


- **A D MCNAUGHT ; A WILKINSON.** Compendium of Chemical Terminology. Blackwell Scientific Publications, 1997
- IUPAC Nomenclature of Organic Chemistry. Blackwell Scientific Publications, 1993
- MICROENCAPSULATION: Methods and Industrial Applications. Marcel Dekker, Inc, 1996
- **C. THIES.** Kirk Othmer Encyclopedia of Chemical Technology Microencapsulation
- **WHITESIDES ; ROSS.** *J. Interface Colloid Sci.,* 1995, vol. 196, 48-59
- Surface modification of inorganic nanoparticles by organic functional groups. Nanoparticle Technology handbook. 2007, 593-596
- *OECD GUIDELINE FOR THE TESTING OF CHEMICALS,* 27 July 1995
- **S. ARCTANDER.** *Perfume Flavors and Chemicals,* 1969, vol. I, II
- Allured's Flavor and Fragrance Materials. Allured Publishing Corp, 2007
- *CHEMICAL ABSTRACTS,* 5989-27-5
- *CHEMICAL ABSTRACTS,* 99-49-0
- *CHEMICAL ABSTRACTS,* 35044-57-6
- *CHEMICAL ABSTRACTS,* 123-35-3
- *CHEMICAL ABSTRACTS,* 543-39-5
- *CHEMICAL ABSTRACTS,* 1118-39-4
- *CHEMICAL ABSTRACTS,* 97-53-0
- *CHEMICAL ABSTRACTS,* 93-28-7
- *CHEMICAL ABSTRACTS,* 501-92-8
- *CHEMICAL ABSTRACTS,* 140-67-0
- *CHEMICAL ABSTRACTS,* 104-46-1
- *CHEMICAL ABSTRACTS,* 3380-34-5

- **L HO TAN TAI.** Formulating Detergents and Personal Care Products A guide to Product Development. AOCS Press
- Surfactant Science Series Liquid Detergents. Marcel Dekker Inc, vol. 67
- Harry's Cosmeticology. CHS Press, 2000
- McCutcheon's, Detergents and Emulsifiers. Allured Publishing Corporation, 1986
- **M R ROSEN ; D BRAUN.** Rheology Modifiers Handbook Practical Uses and Applications. William Andrew Publishing, 2000
- MICROENCAPSULATION Methods and Industrial Applications. Marcel Dekker, Inc, 1996
- **MOAD, GRAEME ; SOLOMON, DAVID H.** The Chemistry of Radical Polymerization. Elsevier, 2006
- **BRAUNECKER, WADE A. ; MATYJASZEWSKI, KRZYSZTOF.** Controlled/Living Radical Polymerization: Features, Developments, and Perspectives. *Progress in Polymer Science,* 2007, vol. 32 (1), 93-146
- *CHEMICAL ABSTRACTS,* 75-91-2
- *CHEMICAL ABSTRACTS,* 51410-72-1
- *CHEMICAL ABSTRACTS,* 125-12-2
- *CHEMICAL ABSTRACTS,* 464-49-3
- *CHEMICAL ABSTRACTS,* 80-54-6
- *CHEMICAL ABSTRACTS,* 470-82-6
- *CHEMICAL ABSTRACTS,* 39255-32-8
- *CHEMICAL ABSTRACTS,* 77-53-2
- *CHEMICAL ABSTRACTS,* 142-19-8
- *CHEMICAL ABSTRACTS,* 93-92-5
- *CHEMICAL ABSTRACTS,* 110-41-8
- *CHEMICAL ABSTRACTS,* 88-41-5
- *CHEMICAL ABSTRACTS,* 91-64-5
- *CHEMICAL ABSTRACTS,* 57378-68-4